# EUROPEAN PATENT APPLICATION

(11) **EP 4 653 520 A1**
(43) Date of publication of application: **26.11.2025**
(21) Application number: 24177036.1
(22) Date of filing: 21.05.2024
(51) Int. Cl.: C12M 1/00

(54) **BIOREACTOR AND GUIDE ELEMENT THEREFOR**

(71) Applicant: Subitec GmbH, 73257 Köngen (DE)
(72) Inventor: Welte, Christian, 7525 S-chanf (CH); Weber, Gregor, 80638 München (DE)
(74) Representative: Kramer Barske Schmidtchen Patentanwälte PartG mbB

(57) **Abstract**

A bioreactor (1) comprising a tank (10) adapted to contain a growth medium (12), and a plurality of guide elements mounted within the tank and comprising at least a first guide element (20), a second guide element (22), and a third guide element (24), wherein the guide elements (20, 22, 24) are provided within the tank (10) such that the guide elements (20, 22, 24) are at least partially immersed in the growth medium (12) when the tank (10) contains the growth medium (12), a first flow chamber (26) and a second flow chamber (28) are defined between the first, the second and the third guide elements and first and second flow paths (30, 32) of the growth medium (12) in a vertical direction (Y) through the respective first and second flow chambers (26, 28) have a waveform shape, a meandering shape, a zigzag shape, an undulating shape or a sinusoidal shape, the guide elements (20, 22, 24) are removably mounted within the tank (10) and are configured to be separable from each other at least when removed from the tank (10).

## Description

### TECHNICAL FIELD

The present invention relates to a bioreactor, in particular a photobioreactor, for cultivating organisms, in particular photoautotrophic organisms, and to at least one guide element to be used in the bioreactor. For example, the present invention relates to a closed photobioreactor for growing photoautotrophic organisms like for example microalgae, cyanobacteria, macroalgae, and some mosses within a liquid growth medium, which is preferably irradiated (illuminated) by one or more artificial light sources.

### BACKGROUND

Generally speaking, bioreactors, in particular photobioreactors, may be implemented as open systems in which, for example, a basin which is open to the environment and exposed to sunlight, contains a growth medium in which the organisms are grown. The present disclosure particularly refers to bioreactors implemented as a closed system. In a closed system bioreactor, a growth medium (also known as culture medium) is circulated within a closed circulation system such that the growth medium is normally not exposed to the environment.

Closed system (photo-) bioreactors avoid water losses, minimize contamination, and enable the relevant factors for effectively growing the organisms (photosynthesis-relevant factors) to be more precisely controlled (regulated). The photosynthesis-relevant factors are, in particular, the exposure of the growth medium to (preferably controllable artificial) light and the input of CO2 into the growth medium. Furthermore, it is important that the growth medium is constantly mixed (agitated) such that the light exposure and the input of CO2 are homogenously distributed over (throughout) the growth medium.

An example of a closed system photobioreactor known in the art is a so-called flat panel airlift photobioreactor (FPA) as manufactured by Subitec GmbH, Stuttgart, Germany. Such photobioreactors are generally formed of two housing shells, which are connected to each other such that a hollow upflow chamber is formed between the two housing shells. The FPA is formed such that an upflow path from bottom to top through the upflow chamber has a waveform (meander) shape. The bottom of the FPA comprises a membrane to introduce CO2 into the upflow chamber. Furthermore, downflow means are provided to allow a downflow (return) of the growth medium to the bottom of the tank. Several FPAs may be arranged as an array/stack for mass cultivation.

In operation, such FPAs are filled with a growth medium. By introducing CO2, a waveform-shaped upward flow of growth medium through the flow path is created (airlift). By irradiating (illuminating) with artificial light, the organisms in the culture will grow (proliferate). The airlift and the waveform-shape enable a high degree of homogenization of the growth medium during the cultivation process.

FPAs are described, for example in WO 2017/055585 A1. Similar photobioreactors are, for example, described in DE 199 16 597 A1 (and its English-language family member US 6,509,188 B1), and US 7,374,928 B2.

### SUMMARY

The interior of closed system bioreactors, including FPAs, must be cleaned from time to time because a biofilm typically forms (grows) on the inside surfaces of the structure holding the growth medium. Known cleaning processes are e.g., rinsing, e.g., with chemicals, and/or hot steam injection (pressure washing). However, the removal of biofilm is difficult to perform in an efficient manner and is thus time consuming.

Furthermore, there is a need to design bioreactors in a space-saving manner.

At the same time, the above photosynthesis-relevant factors should be met with a high degree of precision in order to ensure an efficient production (cultivation).

It is one non-limiting object of the present invention to disclose an improved bioreactor. Furthermore, it is another non-limiting object to disclose an improved a guide element for use in such a bioreactor.

This object is solved by a bioreactor according to claim 1. The object is further solved by a guide element according to claim 17. Further developments are given in the dependent claims.

The bioreactor according to claim 1 comprises at least three guide elements provided within a tank. The three guide elements are provided such that two (fluid) flow chambers are partially formed (defined) within the tank between adjacent ones of the three guide elements. Accordingly, the second guide element, which is provided between the first guide element and the third guide element, partially defines both (fluid) flow chambers.

The guide elements are removable from the tank and separable from each other (at least when they have been removed from the tank).

The above-described structure enables at least two flow chambers to be defined using only three guide elements within the bioreactor in a compact and simple manner because the second guide element is used to partially define both flow chambers. Moreover, the structure enables the guide elements to be easily removed for cleaning of the surfaces defining the respective flow chambers and for cleaning the inside (wall) surfaces of the tank. Also, the structure enables an easy re-mounting of the guide elements in the tank after cleaning.

A bioreactor according to a preferred embodiment is preferably a photobioreactor in which a liquid growth medium may be irradiated (illuminated) with light in order to cultivate photoautotrophic organisms like, for example, microalgae, cyanobacteria, macroalgae, and some mosses. In such an application of the present disclosure, walls of the tank and/or at least a part/portion of the plurality of guide elements is (are) made of a light permeable (transmissible) material such that the light can enter into the (flow) chambers through the tank walls and/or through the guide elements to grow the culture. Thus, at least portions of the walls and/or the guide elements are preferably made of a transparent and/or translucent material that transmits preferably at least 50% of incident light. A preferred material at least for the guide elements or portions thereof is a transparent PP material. Alternatives are other transparent/translucent materials as e.g. glass, PVC, PET-G or PMMA etc.

Preferably, the bioreactor is an airlift photobioreactor, in which the circulation of the growth medium is achieved preferably solely by introducing pressurized gas into a lower portion of the growth medium held in the tank. The uprising pressurized gas results in a corresponding upflow of growth medium.

The tank may be a separate structural member or may be formed (configured) as an integral part of another means (component) of the bioreactor system. The tank may preferably be shaped as a rectangular cuboid. In such an embodiment, the tank has a bottom wall, four side walls (two lateral side walls and a front side wall and a rear side wall) and an upper wall. In alternative embodiments, the tank may have any other shape such as, for example, a cylindrical shape or other type of curved and/or polygonal shape.

The guide elements are preferably formed in a generally flat plate-shape extending in a plane, which is preferably spanned (defined) by (or contains) a vertical direction and a lateral direction, which is perpendicular to the vertical direction. For example, one or more of the guide elements may be formed of a generally flat panel.

In an alternative embodiment (not shown in the drawings), the guide elements may have a zigzag-like shape extending in the vertical direction. Such guide elements preferably extend generally along (in the direction of) a plane, which is preferably spanned (defined) by (or contains) the vertical direction and the lateral direction. Such guide elements extend preferably perpendicular to a plane spanned (defined) by (or containing) the vertical direction and a longitudinal direction, which is perpendicular to the vertical and the lateral directions. Thus, such zig-zag guide elements generally extend in the lateral direction, whereas the zig-zag is formed by the fact that the guide elements do not extend straight in the vertical directions but rather extend with alternating inclinations in the longitudinal direction.

A guide element may be integrally formed (in one-piece) or may be formed with or mounted to more than one member. If a guide element is made of more than one member, the members may be non-separably connected or may be separable (readily detachable) from each other.

Preferably, the guide elements, preferably the first guide element, the second guide element, and the third guide element, each have a similar or identical general shape.

The guide elements are mounted in the tank such that they are completely or partly immersed in the tank, when the tank is filled with the growth medium to a predefined fill level. This includes designs in which some of the guide elements are completely immersed and some of the guide elements are partially immersed.

The guide elements are preferably mounted within the tank by being respectively supported on/by a supporting means. The supporting means is preferably mounted/supported on a tank (preferably rear side) wall. Preferably, the guide elements (at least a first guide element and intermediate guide elements) are supported within the tank without contacting any of the tank walls. However, it is also possible that one or more of the tank walls is (are) contacted by at least one of the guide elements, for example, for guiding the guide element(s), while the guide elements are primarily or completely supported via the supporting means.

Preferably a (last) guide element is provided for forming (defining) a (fluid) flow chamber between a tank (preferably rear side) wall and the (last) guide element, and is formed (configured) such that it contacts or nearly contacts one or more of the tank side walls. Preferably, an additional sealing means such as a rubber lip (seal, gasket) may be provided.

Alternatively, the guide elements may be directly mounted (attached) to at least one tank wall. In this case, for example, the tank wall may comprise insertion grooves into which side edges (rims, flanges) (in the lateral direction) of the guide elements may be inserted. The guide elements may be mounted separately in and/or on a wall of the tank. Alternatively, only one or some of the guide elements may be mounted (attached) to a tank wall (directly or via a supporting means) and the other guide elements may be mounted within the tank via the guide elements mounted to the tank wall.

Preferably, the guide elements are arranged next to each other (side-by-side) in the longitudinal direction such that an array (stack, series) of guide elements is formed, wherein the array (stack) direction is the longitudinal direction.

Preferably, two guide elements arranged next to each other are spaced apart by gap having a first distance, preferably in the longitudinal direction. The first distance (gap) provides that a flow chamber is formed (defined) between the two guide elements.

The flow chamber between two adjacent guide elements is, thus, at least in the longitudinal direction, defined by the two guide elements. Preferably, the flow chamber is an open-ended flow chamber which is at least open at its lower end and its upper end in the vertical direction.

Preferably, for each pair of adjacent (or neighboring) guide elements, between which a flow chamber is formed (defined), the guide elements are connected (to each other) by a spacer means (spacer), the spacer means being provided between the guide elements and providing (defining) the first distance. The spacer means may be formed by/as one or more side connection walls extending at least partly from the bottom to the top (in the vertical direction) of the guide elements (on each of the plates of the guide elements). Preferably, two side connection walls are provided on each guide element and respectively extend adjacent to or on the lateral edges (rims) of the guide element(s). In this case, the (each) flow chamber may be additionally (partially) defined in the lateral direction by the side connection walls.

The connection side walls are preferably integrally formed with the guide elements by performing a thermoforming process. Preferably each guide element comprises two connection side walls on the respective longitudinal side(s) that face (oppose) another (an adjacent) guide element. Preferably, the connection side walls of two guide elements, which together form (define) a flow chamber between them, are formed such that tip ends of opposing connection side walls contact each other when the guide elements are mounted in the tank. Preferably, the connection may be sealed by a sealant or adhesive. Preferably the connection is detachable to facilitate separation of the guide elements for cleaning and maintenance purposes.

Each connection side wall has preferably a first height in the longitudinal direction which is preferably 50% or more of the first distance (gap) between adjacent guide elements in the longitudinal direction. Preferably, the first height of the connection side walls in the longitudinal direction is shorter than a second height of the protrusion portions protruding from the same plate in the longitudinal direction. Preferably, the first distance or the sum of the first heights of two opposing connection side walls contacting each other is larger than the second height of the protrusion portions defining the same flow chamber.

The connection side walls preferably each define a hollow portion (channel) such that a connection side wall cavity is formed therein. The connection side wall cavities may be used as a cooling channel, a wire channel or channel for housing a light emitting means. The connection side wall cavities preferably extend in the vertical direction.

Alternatively, or additionally, the spacer means may be formed at any position and in any shape between two adjacent guide elements. The spacer means may be integrally formed with or on only one or both of the guide elements. Alternatively, the spacer means may be a separate structure that is attached to the guide element.

The guide elements form (define) a flow path within the (each) flow chamber. The flow path includes a vertical extension between a bottom end and a top (upper) end (or vice versa, depending on the flow direction). The guide elements are preferably formed such that the vertical extension (i.e. the distance between the bottom end and the top (upper) end in the vertical direction) of the flow path is shorter than the (minimum, straightest) fluid travel distance through (along) the flow path. Accordingly, the guide elements are preferably formed such that the flow path does not run (extend) straight (directly) between a bottom end and a top (upper) end, but rather in an undulating manner as will be further described below.

Preferably, the (each) flow path extends, in an alternating manner or in sections, both in the vertical direction and also inclined in (towards) the longitudinal direction. Preferably, a waveform shaped flow path is thus formed (defined). However, the present disclosure is not limited to the waveform shape and also encompasses meander shapes or other shapes (e.g., zigzag shape, an undulating shape or a sinusoidal shape) in which the vertical extension of the flow path is shorter than the fluid flow path, as will be further discussed below.

The preferred flow path is, in a generic formulation, thus, longer than a straight flow path. Furthermore, preferably turbulences are created in the flow (growth) medium such that a good mixing (agitation) of the growth medium can be achieved as the growth medium is moved along the flow path.

Alternatively, or additionally, means for hindering (obstructing) a straight flow path such as e.g., flow barriers, may be provided within the flow chambers, which flow barriers create turbulences (but not necessarily a waveform shaped flow path). In such a case, it may also be possible that the guide elements form a generally straight flow path comprising the flow barriers. The flow barriers may be formed integrally with one or both of the guide elements forming the chamber or as separate elements. The flow barriers may also function as a spacer means.

According to a preferred embodiment (claim 2), the first to third guide elements respectively defining at least one two flow chambers comprise protrusion portions (which may also be referred to as flow barriers, baffles, peaks, ridges, flanges, fins, substantially triangular arches having concave sides, etc.) which protrude into the flow chamber(s) defined by the respective guide elements. Furthermore, the protrusion portions of the guide elements are provided such that the protrusion portions protruding in the same chamber are, as viewed in the vertical direction, alternately provided on the two adjacent guide elements defining the flow chamber such that the waveform shaped flow path is formed therebetween.

The protrusion portions may have any shape as long as they block (obstruct) a straight vertical flow path from the bottom to the top and vice versa. Preferably, the protrusions portions are wall portions protruding in the longitudinal direction and extending in the lateral direction, preferably, across the entire width of the flow chamber (or guide element) in the lateral direction. Preferably, the protrusions portions extend laterally between the two connection side walls. Preferably, except for the protrusion portions, the remainder of each guide element is a generally flat (planar) panel extending along the vertical and the lateral directions.

The second height of the protrusion portions in the longitudinal direction is preferably more than 50%, e.g., more than 80%, or e.g., more than 90% of the above first distance (gap) between the guide elements in the longitudinal direction. Thus, the protrusion portions overlap each other as viewed in the vertical direction.

In addition to the extended length of the flow path, which enables an extended time for dissolving (absorbing) gaseous CO2 into the liquid growth medium (degree of utilization) and light irradiation, the protrusion portions also create turbulences in the flow of growth medium such that the growth medium is well mixed when flowing along the flow path.

According to another preferred embodiment (claim 3), each of the guide elements is made of (constituted by) a first plate and a second plate. The first and the second plates are arranged such that planar portions (also denoted as connection portions) thereof are parallel to each other (the protrusion portions are not parallel) and attached (affixed) to each other preferably in (along) a common contact plane (which is preferably spanned (defined) by (or contains) the lateral and the vertical directions). The first and the second plate are preferably fixedly attached to each other, for example, by plastic welding or by an adhesive.

One or both of the plates comprise(s) the protrusion portions which are preferably formed by thermoforming. The plates having one or more protrusion portions may also be denoted as a half-shell.

One or both of the plates comprise(s) the connection side walls (or other type of spacer means).

The thermoformed protrusion portions are preferably generally V-shaped with the bottom tip of the V protruding into the flow chamber (or towards the other guide element defining the same flow chamber). Preferably, flat (straight, planar) connection portions are formed or disposed between the thermoformed protrusion portions. Preferably, the two ends opposite to the bottom tip of the V form the transitions to the flat connection portions.

Preferably, each of the protrusion portions defines a (hollow) protrusion portion cavity which preferably extends in the lateral direction throughout (along) the (entire) respective guide element. Preferably, the (each) protrusion portion cavity is closed by the other plate, which does not form (have) the corresponding protrusion portion.

If protrusion portions are formed on both sides of the guide element, the first plate and the second plate, which together form (constitute) the guide element, are preferably formed symmetrically with respect to a symmetry plane defined by the contact plane of the first plate and the second plate. Accordingly, the first plate and the second plate (forming two half shells) are preferably provided mirror-inverted on each other. In this case, the protrusion portions are provided at the same location in the vertical direction such that opposing protrusion portion cavities overlap to provide a common protrusion portion cavity. Preferably but not limited thereto, the symmetry is also present with respect to the connection side walls.

In an alternative embodiment, at least one of the guide elements is formed by only a single plate. In this case, the single plate may comprise the protrusion portions on one side or on both sides. Also in this case, the protrusion portions may be formed by thermoforming. The protrusion portions may form (on the other side in the longitudinal direction) open grooves. The open grooves may be closed by additional means (structure or component such as a plastic strip) to form a protrusion portion cavity, if desired.

In a preferred embodiment, adjacent flow chambers are formed such that the adjacent flow chambers are symmetrical to each other with a symmetry line within (inside) the (intermediate) guide element. Alternatively, the guide elements are formed such that adjacent flow chambers have the identical design. In this case, cross-sectional views perpendicular to the lateral direction of the adjacent chambers are basically identical and not mirror-inverted.

According to a preferred embodiment (claim 4), the plurality of guide elements comprises more than the above-mentioned first, second, and third guide elements. This means, the bioreactor may comprise in the longitudinal direction (array/stack direction), for example, a first guide element (preferably corresponding to the above first guide element), a plurality of intermediate guide elements (preferably corresponding to the above second and third guide elements), and a last guide element, which include the above first, second, and third guide elements. The last guide element preferably corresponds (is designed the same or similarly) to the first guide element with the difference that the protrusion portions are formed on the opposite side in the longitudinal direction. Preferably, at least ten guide elements are provided in the bioreactor, more preferably between 10 and 100, more preferably between 15 and 50, and more preferably between 20 and 40 such as, for example, 25, 30, or 35.

In this case, one flow chamber (defining one flow path) is preferably formed between each pair of longitudinally adjacent guide elements. For example, in case the first guide element, N intermediate guide elements, and the last guide element are provided, N+1 flow chambers defining respective flow paths are preferably formed. Furthermore, in this case, each of the intermediate guide elements comprises protrusion portions on each (both) of its sides as was described above with respect to the second guide element. Of course, also in this case each of the guide elements is adapted (configured) to be mounted in the tank such that they are completely or partly immersed in the growth medium in the tank when the tank is filled with the growth medium to a predefined fill level. This includes designs in which some of the guide elements are completely immersed and some of the guide elements are partially immersed.

Thus, a simple and modular system for forming a compact bioreactor is provided.

Of course, the present disclosure is not limited to such a design. Instead, if desired, it is also possible to omit some or all of the protrusion portions on some of the guide elements such that a straight flow path is formed in one or more flow chambers between the adjacent guide elements. Also, other designs are possible.

Furthermore, it is noted that the so-called first guide element and last guide element may form the actual first and last guide elements of the bioreactor but may alternatively form the first and last guide elements of a sub-portion (subset) of guide elements within the bioreactor. In other words, the plurality of guide elements (for example four in claim 4) as recited in the claims does not exclude that there are further guide elements or group of guide elements.

According to a preferred embodiment (claim 5), e.g., waveform shaped, meander shaped or zigzag shaped, undulating shaped or sinusoidal shaped flow paths are respectively formed between each pair of adjacent guide elements, preferably of at least a sub-portion (subset) of the guide elements. Thus, a compact bioreactor with a high number of elongated flow paths is created in a simple and modular manner.

It should be noted that bioreactors in which only some (at least two) flow paths are formed in a waveform shape (or any other of the above- or below-mentioned shapes ) are also encompassed by the present disclosure.

According to a preferred embodiment (claim 6), the flow chambers, which have the waveform shape, the meandering shape, the zigzag shape, the undulating shape or the sinusoidal shape (preferably either by providing protrusions or due to the specific shape of the guide elements themselves), are defined as first type upflow chambers. Accordingly, a combination of the subject matter of claim 6 with the subject matter of claim 1 implies that the flow chambers of claim 1 may be first type upflow chambers, which are adapted (configured) to guide an upflow (upward flow) of the growth medium along the flow path.

Furthermore, according to a further and independent preferred embodiment of claim 6, a downflow (downward flow) chamber (channel, passageway) adapted (configured) for guiding a (vertical) downflow of the growth medium is provided between one of the guide elements and an inside surface of the tank. Preferably, the downflow chamber does not have any means (structure, element) for creating a waveform shaped, a meandering shaped, a zigzag shaped, an undulating shaped or a sinusoidal shaped flow path. Instead, the downflow chambers are preferably formed such that a substantially straight (vertical) downflow path is provided. The fluid flow through the downflow chamber may be laminar or substantially laminar.

Furthermore, according to the preferred embodiment of claim 6, the downflow chamber is fluidly connected with the first type upflow chamber(s) such that the growth medium can be circulated through (around) the upflow and downflow chambers. The fluid connection at the upper end portion of the upflow chamber may be preferably formed by an open, upper edge (rim) portion of the guide elements being immersed in the growth medium. Accordingly, the growth medium may simply flow (overflow) upwardly out of the flow chamber(s) and then sideways in the lateral and/or longitudinal direction to a corresponding downflow chamber. Alternatively, the guide elements may comprise holes or parts below the surface of the growth medium for allowing such a sideways fluid flow. The same applies with respect to the lower end, where the growth medium flows from the downflow chamber sideways in the lateral and/or longitudinal direction to the upflow chamber(s).

It is to be noted that the present disclosure is not limited such designs. For example, the disclosure also encompasses an embodiment wherein - different to claim 6 - only one of the flow chambers according to claim 1 is a first type upflow chamber and the other flow chamber is a downflow chamber. In a further embodiment, both flow chambers of claim 1 are defined as upflow chambers and a third flow chamber, as defined, for example, in claim 4, may be the only downflow chamber.

According to another preferred embodiment (claim 7), a first type downflow chamber is defined between the first guide element and a side wall (preferably front side wall) of the tank. In this case, the side wall of the tank is preferably the side wall that is arranged next to the first guide element and is substantially parallel to the first guide element. Accordingly, the first type downflow chamber extends in parallel to the adjacent first type upflow chamber but preferably does not have protrusions. Preferably, the first downflow chamber extends along a plane perpendicular to the longitudinal direction.

By providing a first type downflow chamber at one end side in the longitudinal direction, growth medium, which has flowed upwardly through one or, e.g., more, of the upflow chambers, will be redirected and mixed (agitated) both when flowing from the first type upflow chamber(s) to the first type downflow chamber and when flowing out from the lower end of the first type downflow chamber to the first type upflow chamber(s) again. Furthermore, the flow cross section of the first type downflow chamber may be easily adjusted by adjusting a second distance between the first guide element and the (front) side wall.

According to a preferred embodiment (claim 7), a second type upflow chamber is defined as being present on the opposite side of the (array/stack of) guide elements, i.e., between the third or last guide element and a corresponding opposite side wall (preferably back side wall) of the tank. Preferably, the second type upflow chamber is not defined by protrusion portions such that a straight (vertical) upflow path is defined. Preferably, the last guide element may have a larger extension in the lateral direction than other guide elements in order to seal the second type upflow chamber from the remaining tank volume in the longitudinal direction. The flow cross section of the second type upflow chamber may be easily adjusted by adjusting a third distance between the last guide element and the side wall.

By providing the optional second type upflow chamber, it is possible to enable a portion of the growth medium to flow up at a higher flow speed and flow volume in comparison to the growth medium flowing up through the first type upflow chamber(s) such that an additional mixing (agitation) of the growth medium is achieved.

According to a preferred embodiment (claim 8), a second type downflow chamber is provided. In this preferred embodiment, side connection walls are provided on the lateral end portions of the guide elements at a distance (spacing, gap) from the lateral edges (rims). The inner sides of the side connection walls partially define the (first type up-)flow chambers. On their outer sides, which are facing the corresponding inside surface of a (side) wall surface of the tank, the side connection walls define a second type downflow chamber in the lateral direction. The second type downflow chamber is defined in the longitudinal direction by the outer edge (rim) portions of the guide elements defining the corresponding flow chamber. Accordingly, the second type downflow chamber extends on the lateral outer side of a first type upflow chamber and ensures a mixing of the growth medium.

Preferably, second type downflow chambers are respectively provided on both lateral sides of each flow chamber defined between two guide elements. Preferably, a plurality of (preferably (N+1)x2) second type downflow chambers is provided.

Furthermore, in a preferred embodiment, the lateral edge (rim) portions of the guide elements (or at least one of the guide elements) preferably do not extend to (contact) the inside surface of the tank side wall such that a predetermined lateral gap is present. Accordingly, adjacent second type downflow chambers may be fluidly connected via said gaps which allows a further circulation of the growth medium.

The first type downflow chamber may be combined with the second type downflow chamber. However, bioreactors having only the first type downflow chamber or only the second type downflow chambers are also encompassed by the present disclosure.

According to a preferred embodiment (claim 9), light emitting means for growing (providing light to) the culture are provided within or on one or more of the guide elements. The light emitting means are preferably provided within (inside) the protrusion portions. Preferably, the light emitting means are provided inside the protrusion portion cavity(ies), if present. Alternatively, the light emitting means may be designed to be resistant to (impermeable to and/or compatible with) the growth medium and may be provided on the surface(s) of the guide elements that is (are) disposed within the flow chambers (i.e. so that the growth medium contacts the guide elements).

The light emitting means are preferably LED light sources or fiber optics (optical fibers) and emitters connected to corresponding light sources.

By providing the light emitting on or in, preferably inside the protrusion portion cavity(ies), they can be easily mounted, cleaned, adjusted, and replaced and provide a high irradiation (illumination) output.

Of course, the illumination aspect of the present disclosure is not limited to LED light sources. For example, other light sources may be used, such as fluorescent, halogen or discharge lights. The electrical connection of the light emitting means with corresponding light or energy sources can be made via known plugs/sockets or other types of terminal contacts.

According to a preferred embodiment (claim 10), a pressurized gas chamber is provided/formed in the tank. Preferably, a bottom wall of the pressurized gas chamber is formed (defined) by the bottom wall of the tank. The pressurized gas chamber is, at least on the lower side, preferably partially defined (bounded) by and integrally formed with the bottom wall of the tank. An upper wall of the pressurized gas chamber may be flat, wave shaped, angled or curved. The side walls (in the lateral and longitudinal directions) of the pressurized gas chamber are, where possible, preferably formed integrally with the corresponding side walls of the tank. Alternatively, additional pressurized gas chamber side walls may be provided within the tank. The pressurized gas chamber is supplied with pressurized gas from a pressurized gas source, such as a gas cylinder or a gas source connected to a pump. The pressurized gas may be (e.g., pure) CO2 or a gas mixture containing CO2.

The passage (movement) of gas from the pressurized gas chamber into the interior volume tank is preferably enabled by a membrane or a plurality of micro-openings (e.g., a perforated plate) provided in and/or on the upper wall of pressurized gas chamber. The bioreactor is formed (configured) such that the gas passing from the pressurized gas chamber into the tank rises (vertically flows) into preferably all of the upflow chambers (first type upflow chamber and second type upflow chamber) such that an upflow (lifting, vertically upward movement) of the growth medium is created (induced) by the pressurized gas (i.e. owing to the upward displacement (flow) of the growth medium caused (brought about, induced) by the upwardly rising gas bubbles).

According to a preferred embodiment (claim 11), the upper wall of the pressurized gas chamber is formed (configured) as a partition wall which divides the interior space (volume) defined by the tank walls into an (upper) first tank portion (volume) for containing the growth medium and the guide elements, and a lower second tank portion (volume) for containing and/or supplying the pressurized gas. In this case, the pressurized gas chamber is preferably defined by the side walls of the tank, the bottom wall of the tank, and the partition wall. The partition wall is preferably a flat wall extending preferably perpendicular to the vertical direction.

According to a preferred (alternative) embodiment (claim 12), the pressurized gas chamber is formed (defined, disposed) below the tank. In this case, the bottom wall of the tank and the upper wall of the pressure gas chamber are adapted (configured) to enable pressurized gas within the pressurized gas chamber to enter (penetrate, permeate into) the interior space (volume) of the tank such that the pressurized gas entering the inside (interior volume) of the tank rises into the at least one of the flow chambers, thereby uplifting (upwardly lifting) the growth medium within the respective flow chamber(s). Preferably, the bottom wall of the tank is integrally formed with the upper wall of the pressurized gas chamber. Preferably, the bottom wall of the bottom wall of the tank forms the upper wall of the pressurized gas chamber. Preferably, the membrane and/or opening(s) (perforation(s)) is provided within the bottom wall of the tank.

According to a preferred embodiment (claim 13), the membrane is replaceable, if necessary. For example, the membrane may be formed (configured) as a replaceable insert. Furthermore, in an embodiment utilizing the micro-openings, such micro-openings are preferably provided within a replaceable insert which is adapted (configured) to be inserted into a corresponding insert opening in the bottom wall. The insert may be implemented (configured) as a perforated plate. This structure enables the membrane/micro-openings to be easily replaced in case of corrosion or wear.

According to a preferred embodiment (claim 14), the guide elements are supported on and/or by a supporting means. The supporting means is preferably connected to a removable (outer) wall portion (or removable wall) of the tank such that the guide elements are preferably removable from the tank by simply removing (detaching) the outer wall portion from the tank. The removable wall portion may be configured, e.g., as a door or panel. The removable wall portion may preferably be provided within (or as) a side wall (preferably the back side wall) of the tank. In a preferred embodiment, the removable wall portion forms an entire wall (e.g., the entire back side wall of the four side (vertically extending) walls of the tank).

Alternatively, the removable wall portion may be removed firstly and independently from the array (stack) of guide elements and, afterwards, the guide elements may be removed and separated (detached). In this case, the supporting means for supporting the guide elements is preferably fixed to one of the outer walls of the tank. Alternatively, the supporting means is separate from both of the removable wall portion and the outer walls of the tank and, in a mounted stated, preferably supported within the tank to support the guide elements in a defined manner within the tank. In this case, the supporting means may be removed from the tank together with the guide elements supported thereon. In a further alternative, in case the supporting means is not fixed to the removable wall portion, the removable wall portion is configured as a conventional swing door hinged to one of the outer walls of the tank. Furthermore, in a further alternative, the supporting means may be formed integrally with at least one of the walls of the tank.

In a further alternative, the removable wall/door/hinged door is at least a part of the upper wall or the bottom wall instead of being provided in a or as a side wall. In case the removable wall/door is provided as part of the upper wall of the tank, the guide elements itself or the guide elements supported on a supporting means may be lifted out of the tank after removing the removable wall or opening the hinged door.

Accordingly, in a preferred embodiment, the tank may be formed as a large conventional basin made of, for example, metal or concrete, and the guide elements are hung in the basin. The basin may be closed by a corresponding lid as the removable wall.

Preferably, the supporting means include at least two bars, preferably four bars, for supporting the guide elements thereon. Preferably, the guide elements are hung on or otherwise affixed to or held by the bars.

Preferably, in particular if the removable wall portion is at least a part of a side wall, the removable wall portion is further provided with moving means which may be formed (configured), e.g., as an undercarriage with rollers for easily removing (laterally moving) the removable wall portion.

Preferably, the bars comprise corresponding notches or grooves by which each or at least the first and/or the last of the plurality of guide elements is removably fixed in (at) a predetermined position on the bars. Optionally, the guide elements are fixed on (to) the bars by additional fixing elements, such as, e.g., hooks, latches, screws, adhesive, magnets, etc.

Such a structure enables the guide elements to be easily removed from the tank and then removed (separated) from the bars and from each other to facilitate cleaning of the tank and the individual guide elements.

According to a preferred embodiment (claim 15), at least one downcomer tube (downpipe, downspout, return pipe) is provided. The downcomer tube preferably fluidly connects an upper portion (volume) of the tank with a lower portion (volume) but at different locations with respect to the longitudinal direction and/or the lateral direction such that a further mixing of the growth medium is achieved.

In particular a combination of two or more downcomer systems (e.g., first type downflow chamber, second type downflow chambers and downcomer tube(s)) optimizes a mixing of the liquid (growth medium) and avoids or at least significantly reduces "dead spaces" within the tank.

According to a preferred embodiment (claim 16), heat sink portions are provided in or on side walls of the tank. Preferably, the heat sink portions are provided in or on side walls, the inside surfaces of which define downflow chambers. Preferably, they are provided in or on the lateral side walls. Preferably, heat sink portions are provided in, on or at the lower regions of the corresponding downflow chambers. The heat sink portions may preferably be provided on or adjacent (directly or indirectly contacting) the side wall(s) defining the first type downflow chamber and/or the second type downflow chamber. The heat sink portions may be air cooled or for example, fluidly connected to an external (liquid) cooling (heat exchange) circuit. Preferably, the heat sink portions are pillow plate heat exchangers.

By cooling the downflow chamber in or at a lower region of the downflow chamber, the downflow (downward flow) of growth medium is further accelerated due to the temperature drop, which provides (results in) an improved circulation flow through the bioreactor. Furthermore, the temperature of the growth medium, which is caused to rise due to the irradiation with light and/or by the cultivation process, can be controlled (regulated).

According to a preferred embodiment, the array (stack) direction (longitudinal direction) corresponds to the direction of inserting and removing the array (stack) of guide elements into and out of the tank. With this, the first or (as in the embodiment described in detail below) the last guide element is preferably parallel and adjacent to the removable wall portion.

However, the bioreactor may also be provided (configured) such that the stack (array) direction (which remains the longitudinal direction because the directions are here defined to be fixed with respect to the array or stack) is perpendicular to the insertion direction (vertical orientation remains). In this case, the array or stack may be inserted and removed in the lateral direction. This may be achieved by arranging a first or last guide element in parallel with the side walls of the tank that are perpendicular to the removable wall portion. Thus, in this case, neither the second type upflow chamber nor the first type downflow chamber is defined by the removable wall portion.

According to a preferred embodiment, the removable wall portion may be provided in or as the upper wall of the tank such that the array or stack of guide elements may be vertically lifted from (out of) the tank together with the removable wall portion or afterwards.

According to a preferred embodiment, the first type downflow chamber is provided (defined) between the tank (preferably front) side wall and the first guide element and the second type upflow chamber is provided (defined) between the tank (preferably rear) side wall and the last guide element. It also is possible that the position of the flow chambers may be interchanged.

According to a preferred embodiment, a foam and gas discharge means is provided in an upper wall of the tank or in an upper portion of a side wall above the growth medium level to enable foam to be separated and discharged and to collect superfluous (overflow) pressurized gas. The space (volume) in the tank above the growth medium level is preferably kept at a pressure of 0.5 to 1.5 bar in order to reduce foaming.

The tank is preferably made of materials that are durable with respect to pressure, light and growth medium. Preferably, metal, preferably stainless steel, or resin or polymer (PE) or mineral materials as concrete or glass materials, or alternative hybrid materials as reinforced materials may be used.

The guide elements are preferable made of e.g., polypropylene or one or more other shapeable polymer materials, which are durable with respect to pressure, light and growth medium and which have suitable light transmissivity to permit light from the artificial light source(s) to transmit therethrough.

Preferably, the guide elements, in particular the protrusions, are made by thermoforming (i.e., heating the material (here, preferably a polymer), and then pressing/stamping it between one or two dies of a molding die). However, other manufacturing methods may also be utilized, such as casting or injection molding.

The tank preferably further comprises a discharge opening. Furthermore, preferably hot steam for cleaning may be introduced via the pressurized gas chamber into the tank or via a separate steam introduction opening. In particular hot steam cleaning is preferably performed after the guide elements are reinstalled in the tank and after the removable wall has been re-mounted. In this case, the hot steam cleaning can be used for sterilization of the bioreactor for the next production cycle. Preferably sterilization is performed with hot steam at about 121 degrees Celsius and a pressure of 2 bar for 15 minutes.

In order to control the operation, the bioreactor further preferably comprises measuring devices for measuring pH, temperature and density of the growth medium, and electronic control devices (e.g., a computer) for controlling the temperature and supply of pressurized gas. Additional peripheral devices are a compressor, a blower (fan) for cooling, different tanks, and a hot steam generator.

Additional aspects of the present disclosure include, but are not limited to:

A bioreactor, preferably a photobioreactor, preferably comprising
a tank adapted (configured) to contain a growth medium, and
a plurality of guide elements provided (disposed) within the tank next to each other (side-by-side) as an array or a stack in a longitudinal direction, which is perpendicular to a vertical direction, the plurality of guide elements comprising in the following sequence in the longitudinal direction, a first guide element, at least two intermediate guide elements, and a last guide element,
wherein
the guide elements are provided (disposed) within the tank such that the guide elements are at least partially immersed in the growth medium when the tank contains the growth medium,
the first, the intermediate and the last guide elements are arranged next to each other in the longitudinal direction each separated by a distance (spacing, gap) such that a plurality of flow chambers is respectively formed (defined) between guide elements which are arranged next (adjacent) to each other,
the guide elements are formed (defined) and provided (disposed) such that respective flow paths of the growth medium in a vertical direction through the respective flow chambers have a waveform (wave-like or waveform-like) shape, a meandering shape, an undulating shape, an oscillating shape, a sinusoidal shape, or a zigzag shape or combinations thereof, or such that respective flow paths of the growth medium in a vertical direction through the respective flow chambers have lengths which are longer than the vertical extensions (lengths) of the flow paths,
the guide elements are removably mounted within the tank such that the inside surfaces of the tank can be (easily) cleaned when (after) the guide elements have been removed, and
the guide elements are configured to be separable from each other when removed from the tank such that the surfaces of the guide elements forming (defining) the flow chambers in the mounted state can be (easily) cleaned.

The previously mentioned features (including the embodiments of claims 2 to 15) may be combined with the subject-matter of the above aspect.

As it is clear from claims 17 and 18, the disclosure also encompasses the guide elements as such, i.e., without being mounted in a bioreactor.

### BRIEF DESCRIPTION OF THE DRAWINGS

Fig. 1 shows a schematic and simplified cross-sectional view perpendicular to a lateral direction of a bioreactor according to a first embodiment,
Fig. 2 shows a schematic and simplified cross-sectional view perpendicular to the lateral direction of a first guide element,
Fig. 3 shows a schematic and simplified cross-sectional view perpendicular to the lateral direction of a second or intermediate guide element according to a first embodiment,
Fig. 4 shows a schematic and simplified three-dimensional view of three stacked guide elements,
Fig. 5 shows a schematic and simplified cross-sectional view perpendicular to a vertical direction of the bioreactor according to the first embodiment,
Fig. 6 shows a schematic cross-sectional view perpendicular to a longitudinal direction of the bioreactor according to the first embodiment,
Fig. 7 shows a schematic three-dimensional view of the opened tank of the bioreactor, and
Fig. 8 shows a schematic three-dimensional view of the removed wall portion of the bioreactor.

### DETAILED DESCRIPTION

Embodiments of the disclosure will be further described in the following with reference to the drawings. The drawings include an indication of a coordinate system wherein a longitudinal direction is denoted as X, a lateral direction is denoted as Z and a vertical direction (which preferably corresponds to the gravity direction) is denoted as Y.

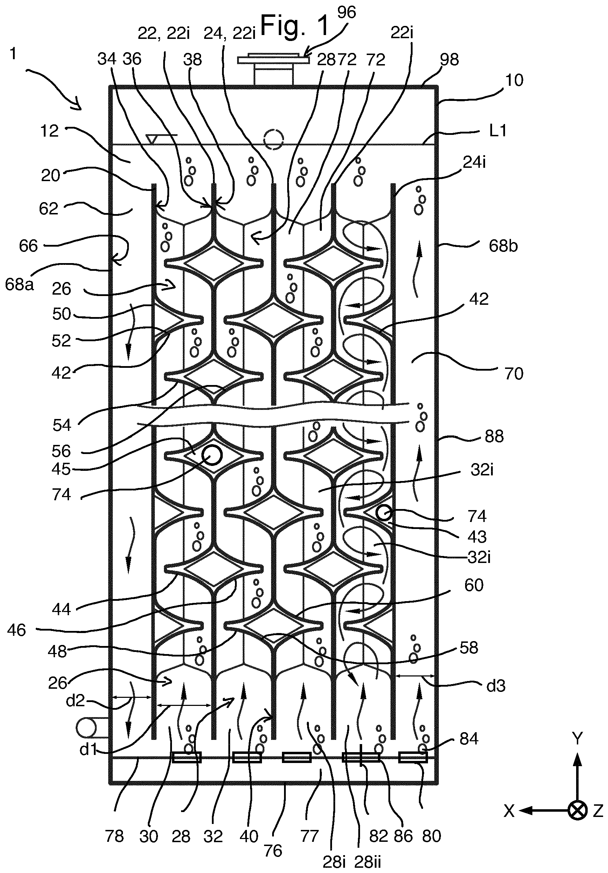

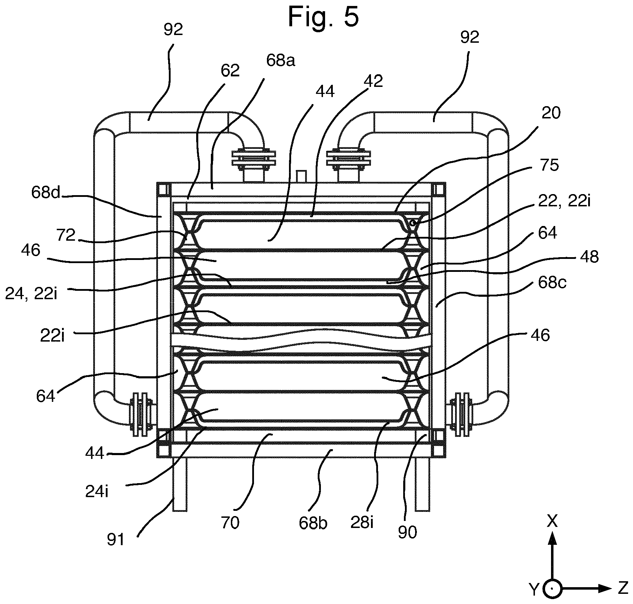

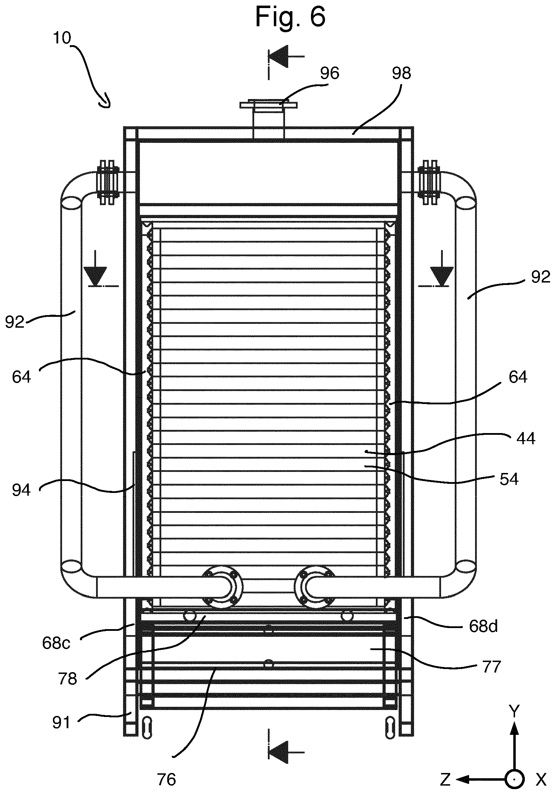

A first embodiment of a bioreactor 1 is shown schematically in simplified way in Figs. 1, 5 and 6. Fig. 6 indicates where the vertical cross-sectional view of Fig. 1 and where the horizontal cross-sectional view of Fig. 5 have been taken. To simplify illustration of significant portions of the first embodiment in the Figures, the drawings are not fully consistent. For example, Figs. 6 to 8 are less simplified than Figs. 1 to 4. Fig. 5 shows a broken view with a broken (wavy) line that is not reflected in Fig. 1.

The bioreactor 1 comprises a tank 10. The tank 10 is shown filled with a growth medium 12 at least up to a certain level L1 in vertical direction Y. For example, the level L1 may define a volume that is at least 70%, preferably at least 80% or at least 90%, of the total volume of the interior space (volume) of the tank 10. Inside the tank, a plurality of guide elements 20, 22, 24 is provided. When the tank 10 is filled with growth medium 12 up to the level L1, the guide elements 20, 22, 24 are at least partially immersed, preferably nearly completely or completely immersed, in the growth medium 12. The guide elements include at least a first guide element 20, a second guide element 22 and the third guide element 24 arranged next (adjacent) to each other (one after another, e.g., in sequence or succession) in longitudinal direction X, which is perpendicular to the vertical direction Y. Each of the guide elements 20, 22, 24 is formed (shaped, configured) as a fluid guiding panel having a partially flat shape (with protrusions projecting from the flat portions thereof) extending in a plane parallel to a plane spanned by (containing) the vertical direction Y and lateral direction Z, which is perpendicular to the longitudinal direction X and to the vertical direction Y. In other words, each guide panel 20, 22, 24 extends perpendicular or at least substantially perpendicular to the longitudinal direction X and thus preferably extends parallel to the vertical direction Y. The guide panels 20, 22, 24 preferably extend parallel to each other.

A first (fluid) flow chamber (channel, passageway) 26 is formed (defined) between the first and second guide elements 20, 22. A second (fluid) flow chamber (channel, passageway) 28 is formed (defined) between the second and third guide elements 22, 24. Thus, both flow chambers 26, 28 are defined or bounded in part by the second guide element 22. The thicknesses (widths) of the flow chambers 26, 28 in the longitudinal direction X are defined by distance d1 between adjacent ones of the guide elements in the longitudinal direction X. The distance d1 is preferably the sum of the first heights h1 of two connection side walls contacting each other.

Within the flow chambers 26, 28, respective waveform shaped flow paths 30, 32 are defined. Herein, the shape or spatial contour of the fluid flow paths 30, 32 along the vertical direction, as defined by the guide elements 20, 22, 24, may be alternately characterized, e.g., as zigzag, undulating, wave-like (waveform shaped), oscillating or sinusoidal. The growth medium flows along the flow paths 30, 32 when the bioreactor is in operation (see also the exemplary arrows in the exemplary flow path 32i in Fig. 1).

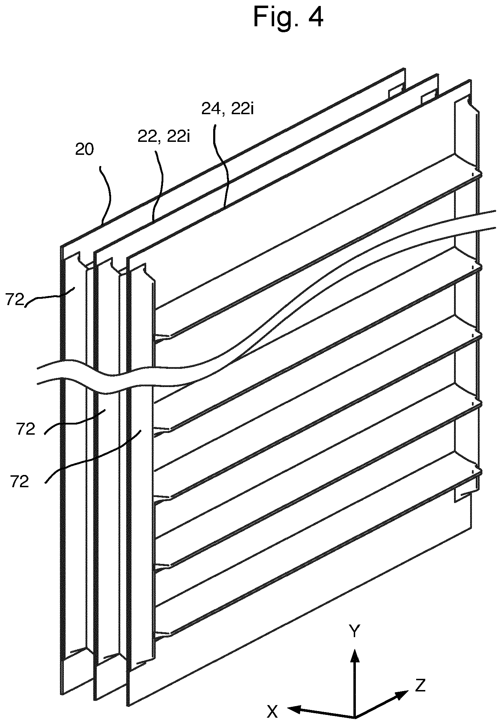

The waveform shape of the flow paths 30, 32 is achieved by providing means (e.g., flow barriers, baffles, protrusions, etc.) for preventing, obstructing or blocking a straight (linear) flow of the guide medium 12 in the vertical direction Y. In the present embodiment, such means are or comprise protrusion portions 42, 44, 46, 48 that protrude (project) horizontally in the longitudinal direction X (as can be seen, e.g., in Figs. 1-3) and extend over (across) the width (preferably the entire width or at least substantially the entire width) of the respective guide element in the lateral direction Z (as can be seen, e.g., in Fig. 4).

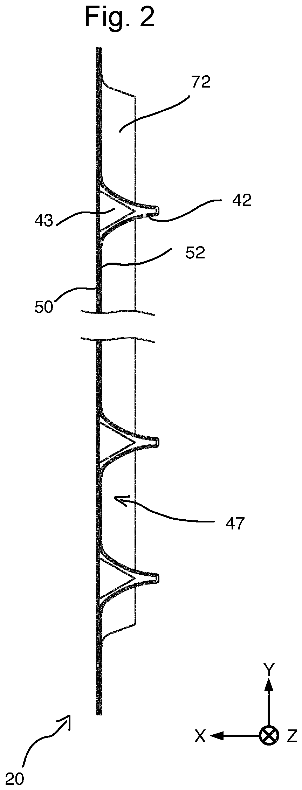

The first guide element 20, which is shown in more detail in Fig. 2, has a plurality of protrusion portions (protrusions, baffles, peaks, ridges, flanges, fins, substantially triangular arches having concave sides) 42, three of which are shown in Figs. 1 and 2. Each protrusion portion 42 is provided on the (longitudinal direction) side of the first guide element 20 which faces the second guide element 22, so that the facing sides of the guide elements 20, 22 define the first (fluid) flow chamber 26. Accordingly, the protrusion portions 42 protrude in the longitudinal direction X (with negative sign).

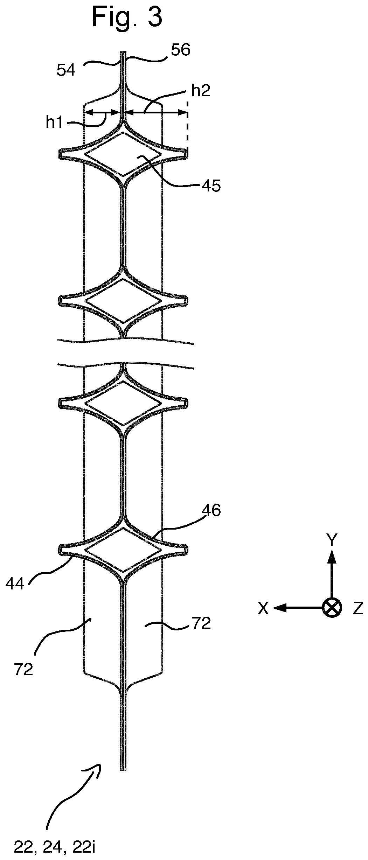

The second guide element 22, which is shown in more detail in Fig. 3 and is also described as (considered to be) an embodiment of a guide element according to the present disclosure, has a plurality of first protrusion portions 44 and a plurality of second protrusion portions 46 on the respective sides, four of each are shown on each side in Figs. 1 and 3 (a more realistic view is shown in Fig. 6, where a plurality of protrusion portions 44 is shown). Each first protrusion portion 44 is provided on the (longitudinal direction) side of the second guide element 22 which faces the first guide element 20. Accordingly, the first protrusion portions 44 protrude in the longitudinal direction X (with positive sign).

In order to achieve the waveform shape (or e.g. the meandering shape, the undulating shape, the oscillating shape, the sinusoidal shape, or the zigzag shape or combinations thereof, the protrusion portions 42 on the first guide element 20 and the protrusion portions 44 on the second guide element 22 are, as viewed along the vertical direction Y through the flow chamber 26, alternately provided (defined) on the guide elements 20, 22, i.e. disposed at alternating positions along the vertical direction Y. Furthermore, each protrusion portion 42, 44 preferably has a second height (length) h2 in the longitudinal direction X which is more than 50% of the distance d1 between the guide elements 20, 22 such that the protrusion portions overlap each other when viewed in the vertical direction (see Fig. 5). For example, the second height h2 is preferably at least 60% of the first distance d1, such as at least 70% of the first distance d1 or at least 75% or at least 80% of the distance d1. Furthermore, the second height h2 is preferably less than 95% of the distance d1, such as less than 90% of the distance d1 or less than 85% of the distance d1. Ranges for the second height h2 may be arbitrarily defined based on any of the preceding upper and lower limits. The second height h2 is defined as the distance (length) in the longitudinal direction X from a base portion of each protrusion portion 42, 44, 46, 48 that is colinear with the flat portions of the guide elements to the apex (peak, top, crest) of the protrusion portion 42, 44, 46, 48 in the longitudinal direction X.

The second (fluid) flow chamber 28 is substantially mirror-inverted to the first (fluid) flow chamber 26 with a symmetry line lying in (along, colinear with) the second guide element 22. Thus, the second guide element 22 itself has a mirror symmetrical shape with respect to the symmetry line. Accordingly, the second guide element 22 comprises the second protrusion portions 46 protruding opposite (in the longitudinal direction X) to the first protrusion portions 44 towards the third guide element 24. Furthermore, the third guide element 24 has (first) protrusion portions 48 protruding towards the second guide element 22 in a mirror-inverted way with respect to the protrusion portions 42 of the first guide element 20. In other words, the protrusion portions 42, 48 extend in a mirror-symmetric way when the vertical extension of the second guide element 22 is considered to be the symmetry line or plane for first and third guide elements 20, 24.

The third guide element 24 has, similar to the second guide element, protrusion portions on both sides in the longitudinal direction because another (third) flow chamber 28i is formed (defined) on the side opposite to the second guide element 22 of the third guide element 24. Guide elements, which have protrusion portions on both sides, i.e. similar to the second and third guide elements 22, 24, will also be called intermediate guide elements 22i in the following description and in the claims (see also Fig. 3).

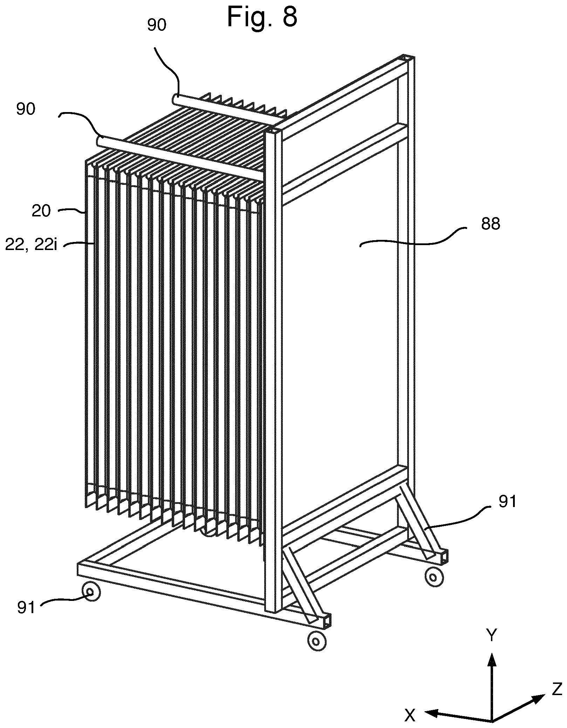

In addition to the second and third guide elements 22, 24, the bioreactor 1 shown in Fig. 1 has, in the longitudinal direction X, a fourth guide element that serves as another intermediate guide element 22i, and a fifth guide element that serves as a last (longitudinally outermost) guide element 24i of the array (stack, sequence, alignment) of guide elements. Please note that Fig. 1 shows only five guide elements in an exemplary manner, whereas in practice more guide elements preferably may be used, as can be seen in Fig. 8. This can also be seen from Fig. 5, wherein the two wavy lines are intended to indicate that more than the guide elements, which are shown, may actually be present. Corresponding wavy lines were omitted in Fig. 1 for clarity reasons. (The wavy lines shown in Fig. 1 are intended to indicate that the guide elements 20, 22, 22i, 24, may be longer, and thus have additional protrusion portions, in the vertical direction Y than is actually shown in Fig. 1. That is, additional protrusion portions can be seen e.g., in Figs. 4 and 6.)

In Fig. 1, an uneven number of (i.e. five) guide elements is shown, which means that the last guide element 24i is provided (shaped, configured) in a mirror-inverted manner with respect to the first guide element 22. In other words, when the vertical extension of the middle intermediate guide element 22i is considered to be the symmetry line for first and last guide elements 20, 24i, the protrusion portions 42 of the first and last guide elements 22, 24i extend in a mirror-symmetric way. Thus, similar to the first guide element 20, the last guide element 24i also has protrusion portions only on the side facing the adjacent intermediate element 22i.

In the first embodiment, each guide element is formed of (comprises, is constituted by) two plates which are attached to each other. Preferably, the two plates are affixed to each other by an adhesive or plastic welding (fusing).

Specifically, the first guide element 20 comprises a planar (flat) first plate 50 and a second plate 52 (see also Fig. 2). The second plate 52 comprises the protrusion portions 42 and planar (flat) portions 47 respectively extending between adjacent ones of the protrusion portions 42. The planar portions 42 extend in parallel to the first plate 50 and connect the protrusion portions 42 in the vertical direction Y. The planar portions 47 flushly contact the planar first plate 50 when the two plates 50, 52 are affixed to each other.

The protrusion portions 42 are preferably formed by performing a thermoforming process such that (first type) protrusion portion cavities (hollows) 43 are created between the first plate 50 and the protrusion portions 42 of the second plate 52.

The last guide element 24i is formed in the same or similar way but in a mirror-inverted manner.

The intermediate guide elements 22i (or the second and third guide elements 22, 24) comprise a first plate 54, 58 and a second plate 56, 60, which have the respective protrusion portions 44, 46, 48 extending in opposite directions formed (defined) thereon (see also Fig. 3). Thus, a (second type) protrusion portion cavity 45 is formed between each pair of oppositely extending (projecting) first protrusion portion 44, 46 and second protrusion portion 48.

Thus, four waveform shaped fluid flow paths are formed (provided) within the flow chambers 26, 28, 28i respectively defined between each two adjacent ones of the guide elements. In the first embodiment, these (fluid) flow chambers defined between the guide elements will be referred to as first type upflow chambers (i.e. chambers, channels or passageways, in which the growth medium is intended to flow vertically upwardly). Therefore, when the bioreactor 10 is operating, the growth medium flows upward through the first type upflow (rising, vertically-extending) chambers 26, 28, 28i.

Because the growth medium 12 has to be circulated within the tank 10, it is necessary to provide at least one passageway for a downflow (vertically downward flow) of the growth medium. The bioreactor 1 according to the first embodiment has a first type downflow chamber (channel, passageway) 62 and a second type downflow chamber (channel, passageway) 64 for enabling the growth medium to flow downward (see Fig. 5).

As can be seen in Fig. 1, the first type downflow chamber 62 is formed (defined) between the first guide element 20 and a corresponding (opposing) inside surface 66a of the front side wall 68a of the tank 10. The first type downflow chamber 62 extends (spans) a distance d2 in the longitudinal direction X from the corresponding inside surface 66a of a front side wall 68a of the tank 10 to the flat first plate 50 of the first guide element 20 (see also Fig. 5).

Because the guide elements 22, 22i, 24i are completely immersed in the growth medium 12 in the embodiment shown in Fig. 1, growth medium 12 can flow from (over) open, upper end portions (edges) of the first type upflow chambers 26, 28, 28i to the first type downflow chamber 62. After flowing downward through the first type downflow chamber 62, the growth medium 12 can flow to open, lower end portions (edges) of the first type upflow chambers 26, 28, 28i and can then flow again upwards through the first type upflow chambers 26, 28, 28i.

Furthermore, the bioreactor 1 according to the first embodiment also comprises a second type upflow chamber 70 (see Figs. 1 and 5) which is formed (defined, shaped) in the same way as the first type downflow chamber 62 but on the opposite side of the array (stack) of guide elements.

In particular, the second type upflow chamber 70 is formed (defined) between the last guide element 24i and a corresponding (opposing) inside surface 66b of the rear side wall 68b of the tank 10. The second type upflow chamber 70 extends (spans) a distance d3 in the longitudinal direction Y from the corresponding inside surface 66b of a rear side wall 68b of the tank 10 to the flat plate of the last guide element 24i.

Thus, the first type downflow chamber 62 and the second type upflow chamber 70 are formed (defined) at opposite positions (on opposite sides) in the longitudinal direction X on both ends (sides) of the array (stack) of guide elements.

Second type downflow chambers 64 (described in more detail below) are generally formed on the lateral sides of the array (stack) of guide elements and defined by corresponding surfaces 66c or 66d of lateral side walls 68c or 68d of the tank 10.

Because the guide elements 20, 22, 22i, 24 are removably provided (held) within the interior space (volume) of the tank 10, the guide elements 20, 22, 22i, 24 are supported within the tank with a clearance fit or even without directly contacting any inside surface of the walls of the tank 10, as can be understood by viewing Figs. 1, 7 and 8 together.

To create a well-defined and strong flow of the growth medium through the respective (fluid) flow chambers, it is preferred that the respective (fluid) flow chambers are separated (isolated, partitioned) from each other. In particular, it is preferred that the upflow chambers are separated (isolated, partitioned) from the downflow chambers except for the fluid connection of the chambers at the upper end portions and at the lower end portions to form a closed loop. Therefore, in particular there should be no direct fluid connection in regions between the upper end portions and the lower end portions of the respective (fluid) flow chambers.

The first type upflow chambers 26, 28, 28i are defined (bounded, circumscribed) in the longitudinal direction X by the guide elements. In order to also define (bound, circumscribe) the first type upflow chambers 26, 28, 28i in the lateral direction Z, the bioreactor 1 further comprises connection side walls 72 (see also Fig. 4). The connection side walls 72 are provided (disposed) between each pair of adj acent guide elements 20, 22, 22i, 24, 24i to forming a first type upflow chamber 26, 28, 28i at the lateral edge (rim) portions of the guide elements 20, 22, 22i, 24, 24i and have a longest extension (length) in the vertical direction Y. Specifically, the connection side walls 72 are integrally formed by thermoforming the flat plates of both of the adjacent guide elements 20, 22, 22i, 24, 24i and connect the adjacent guide elements 20, 22, 22i, 24, 24i such that the corresponding first type upflow chamber is defined also in the lateral direction Z.

The second type downflow chambers 62 are formed between the connection side walls 72 and corresponding inside surfaces 66c or 66d of the lateral side walls 68c or 68d of the tank 10.

Specifically, in the present embodiment, the connection side walls 72 extend (span) a distance to the lateral edge (rim) of the corresponding guide element 20, 22, 22i, 24, 24i such that the second type downflow chamber 64 is at least partly defined also by the corresponding guide elements 20, 22, 22i, 24, 24i in the longitudinal direction X.

In particular, the connection side walls 72 are formed by thermoforming the edge (rim) portion of the corresponding guide element 20, 22, 22i, 24, 24i, thereby resulting in a cross-section perpendicular to the vertical direction that is a V-shape or truncated cone shape, where the top of the shape has a greater distance to (is farther from) the edge than the base, as can be seen in Fig. 5 along the left and right sides of the bioreactor.

Furthermore, the bioreactor 1 according to the first embodiment comprises LED stripes (strips) as light emitting means 74, which are provided inside some or all (in Fig. 1 only one is shown for the sake of simplifying the illustration) of the (first and second type) protrusion portion cavities 43, 45. Preferably, the protrusion portion cavities 43, 45 are sealed (e.g., in a water-tight manner) from the surrounding environment, such that the stripes are protected (shielded, isolated) from the growth medium. Corresponding seals or gaskets (necessary seals therefor) are not shown for the sake of simplifying the illustration. Furthermore, the connection side walls 72 are preferably formed (shaped) such that a connection side wall cavity 75 (shown in a simplified manner only in Fig. 5) is provided therein. The connection side wall cavities 75 are designed and used to receive (contain, hold) in a similar way to the protrusion portion cavities additional LED stripes (strips) and/or to receive (contain, hole) one or more power cables for distributing energy (conducting current) to the light emitting means 74.

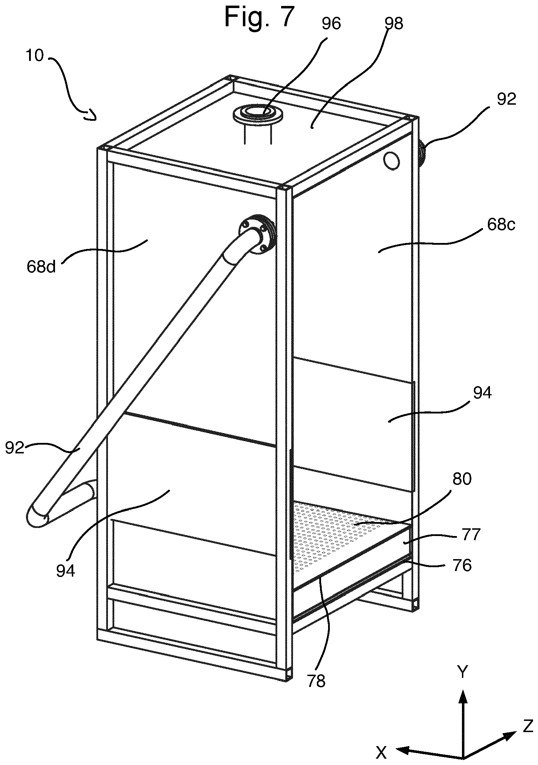

Furthermore, the tank 10 of the bioreactor 1 according to the first embodiment comprises a bottom wall 76 which integrally forms a bottom wall of a pressurized gas chamber 77, as can be seen in Fig. 7. Furthermore, a partition wall 78 is provided and divides the interior volume of the tank 10 into an (upper) first tank portion, which is to be filled with the growth medium 12 and which contains the guide elements, and a (lower) second tank portion, which forms the pressurized gas chamber 77. As can be seen in Fig. 1, the partition wall 78 is formed such that a pressurized gas 84, in particular CO2 (carbon dioxide) or a gas mixture containing CO2, can be introduced therethrough into interior space (volume) of the first tank portion.

The pressurized gas chamber 77 may be supplied with or filled with pressurized gas 84 from a conventional pressurized gas source, such as a pressurized gas cylinder (not shown). The pressurized gas 84 is introduced into the tank such that the pressurized gas 84 rises from the bottom wall 76 into the (preferably both of the first type and second type) upflow chambers 26, 28, 28i, 28ii, 70 but not into the (first and second type) downflow chambers 62, 64. Due to this design, when the pressurized gas 84 rises in the upflow chambers 26, 28, 28i, 28ii, 70, the pressurized gas 84 acts to uplift (upwardly push) the growth medium 12 within the upflow chambers 26, 28, 28i, 28ii, 70, thereby creating (inducing) an upflow (upwardly moving current) of growth medium 12 through the upflow chambers 26, 28, 28i, 28ii, 70. Thus, this mechanism achieves (effects, generates) a circulating flow of the growth medium 12 upwardly through the upflow chambers 26, 28, 28i, 28ii, 70 and then downwardly through the downflow chambers 62, 64.

To enable the pressurized gas 84 to penetrate through the partition wall 78, the partition wall 78 comprises a membrane 80 and/or openings (preferably, micro-openings) 82 (see Figs. 1 and 7). Preferably, the micro-openings (perforations) are formed in a corresponding insert 86 (Fig. 1), which is preferably configured (adapted) to be removably inserted into a corresponding insert opening in the bottom wall 76. The membrane 80 and/or the insert 86 are formed such that they can be replaced if necessary (for example, in case of corrosion, wear, breakage, etc.).

In the first embodiment, the tank 10 comprises a removable (detachable) wall portion 88 (see Fig. 8). In the present embodiment, the removable wall portion 88 is formed (constituted) by the rear side wall 68b, which is provided adjacent to the last guide element 24i. The removable wall portion 88 is, for example, detachably mounted (joined in a water-tight manner) to the tank 10 by screws and sealed by a seal or gasket (both not shown) in a conventional manner. The removable wall portion 88 is preferably adapted (configured) to close (enclose, seal) the above-described upper first tank portion (volume), which will hold the growth medium 12.

The inner side of the removable wall portion 88 comprises supporting means 90 for supporting (holding, fixing) the plurality of guide elements 20, 22, 22i, 24, 24i. For example, the supporting means 90 may be, e.g., four bars 90 that extend in parallel in the longitudinal direction X and are provided at or proximal to corner (or edge) portions of the removable wall portion 88. The guide elements 20, 22, 22i, 24, 24i are, respectively, supported (held, fixed) by the bars 90. The supporting means 90 may be constituted in a variety of ways known to skilled persons in the art and are not limited to the four bars 90 of the present embodiment. For example and without limitation, fewer or greater than four bars 90 may be utilized. The bars 90 may be wider, e.g., plate shaped than the rod-like bars 90 shown in Fig. 8.

Furthermore, the present embodiment is designed such that the guide elements 20, 22, 22i, 24, 24i can be removed from the tank 10 simply by removing (detaching, separating) the removable wall portion 88 from the tank 10.

The removable wall portion 88 further preferably includes moving means 91 (see particularly Fig. 8). In the present embodiment the moving means 91 is formed (configured) as an undercarriage with rollers (wheels) for easily moving the assembly of the removable wall portion 88 and the guide elements 22, 22i, 24 on a surface, e.g., when removing the removable wall portion 88 from the tank 10 and subsequently re-attaching the removable wall portion 88 to the tank 10. The moving means 91 may be constituted in a variety of ways known to skilled persons in the art and are not limited to the undercarriage and four rollers of the present embodiment. For example and without limitation, fewer or greater than four rollers may be utilized. The moving means 91 may be designed to receive the forks of a mechanical lifting device such as a lowlift truck or forklift, thereby omitting rollers or other types of wheel structures.

Furthermore, in the first embodiment, one, some or all of the guide elements preferably comprise(s) either one or more electric sockets (not shown) which is (are) connectable to an electric plug or plugs (not shown) provided on at least one of the bars 90, or one or more electric plugs which is (are) connectable to an electric socket or electric sockets provided on or in at least one of the bars 90. The electric plug(s) or electric socket(s) is (are) connected to a power source via appropriate wiring.

Furthermore, two downcomer tubes (downpipes, down spouts, return pipes) 92 optionally may be provided. The downcomer tubes 92 are, on the upper side, in fluid connection with the interior volume of the tank at upper portions of lateral side walls of the 68c and 68d of the tank 10 close to the upper end of the of the second type upflow chamber 70. On the lower side, the downcomer tubes 92 are in fluid connection with the interior volume of the tank 10 at lower portions of the longitudinal side wall (front wall) 68a adjacent to the first type downflow chamber 62.

The lateral side walls 68c and 68d further comprise lower region heat sink portions (heat sinks) 94 (see Fig. 6 and 7, not shown in Fig. 1). Preferably the heat sink portions 94 may be formed (constituted) as conventional (e.g., metallic or thermally conductive) cooling plates, fins and/or ribs provided to increase the heat exchange between the inside surface and the outside surface such that the downflowing growth medium is cooled when the growth medium passes along the heat sink portions 94. Alternatively, or additionally, a fluid cooling component, such as a coolant fan, liquid coolant tubes, Peltier element, etc., may be provided to actively cool the heat sink portions 94 during operation of the bioreactor 1 (e.g., in response to detection of the growth medium 12 exceeding a first predetermined temperature). More preferably, the heat sink portions 94 are pillow plate heat exchangers. In addition or in the alternative, a fluid heating component, such as a heating fan, liquid heating tubes, a Peltier element, etc., may be provided to heat the heat sink portions 94 during operation of the bioreactor 1 (e.g., in response to detection of the growth medium 12 falling below a second predetermined temperature that is lower than the first predetermined temperature). In this way, the temperature of the growth medium during operation of the bioreactor 1 may be suitably regulated to maintain the temperature of the growth medium in an optimal temperature range for the organism(s) being cultivated therein.

A foam and gas discharge means (port, discharge opening, aperture) 96 is provided in an upper wall 98 of the tank 10, as can be seen in Figs. 6 and 7. The foam and gas discharge means 96 is adapted (configured) to separate foam, which may build up due the airlift from (agitation of) the growth medium, and to discharge the foam to a not shown foam collector. Furthermore, it is adapted (configured) to collect superfluous (overflow) pressurized gas which has risen (vertically passed) through the growth medium. In order to reduce foam generation, the space (gaseous volume) above the top surface of the growth medium is preferably kept at a pressure of 0.5 to 1.5 bar.

The bioreactor can be manufactured and mounted as follows:
The walls of the tank 10 including the pressurize gas chamber 77 may be made of welded stainless-steel plates. The removable wall 88 may be a stainless-steel plate that is mountable on the tank 10 by e.g., screws or quick release fasteners (e.g., manually- or electronically-actuatable clamps, latches, etc.). The bars 90 may be welded to the inside surface of the removable wall 88.

The guide elements 20, 22, 22i, 24, 24i may be made from polypropylene or other type of plastic (polymer) plates 50, 54, 58, 52, 56, 60 which are then thermoformed to form two half shells (first plate and second plate). The plates are connected by adhesive or plastic welding (fusing) to form the respective guide elements 20, 22, 22i, 24, 24i.

The light emitting means 74 and/or wires and connectors are then mounted (accommodated, held) inside and/or on the guide elements 20, 22, 22i, 24, 24i.

To initiate operation, the bioreactor 1 is filled with growth medium 12 up to a defined upper level L1 so that the guide elements are preferably completely immersed in the growth medium 12. Then light irradiation (illumination) and pressurized gas supply are started in order to facilitate the growth of the culture (e.g., one or more photoautotrophs) that has been inoculated into the growth medium 12. The growth medium 12 is circulated and mixed within the bioreactor 1 by the supply of pressurized gas into the tank 10.

For harvesting or cleaning, the growth medium 12 is discharged (drained) via a discharge means (port, opening, aperture) such as a discharge (drainage) pipe. Then, for cleaning, the removable wall 88 is removed (detached) from the tank 10 and the guide elements 20, 22, 22i, 24, 24i are removed (separated, detached) from each other. Then, the tank 10 and the guide elements 20, 22, 22i, 24, 24i are cleaned e.g., by high pressure water jet cleaning (pressure washing). Furthermore, an overflow pipe may also be provided in order to maintain the desired growth medium level.

According to an alternative (modified) embodiment, the pressurized gas chamber is provided below the tank. In this case, a top wall of the pressure gas chamber is preferably integrally formed with or connected to a bottom wall of the tank and the bottom wall of the tank preferably comprises the membrane or openings as e.g., an insert with perforations.

If these definitions (modifications) of the alternative embodiment are applied to Fig. 1, the wall denoted by reference sign 78 may be referred to as the bottom wall of the tank and the chamber denoted by reference sign 77 may be referred to as the pressurized gas chamber.

According to a further alternative embodiment (not shown), a conventional (swing) door for opening the tank and removing the guide elements is provided instead (or as) the removable wall. Obviously, such a swing door is connected to one of the remaining walls via a hinge and can be closed in a tight manner by a seal or gasket and closed by a corresponding lock mechanism. The swing door may be provided in one of the side walls and/or in the top/bottom walls. As in the above embodiment, supporting means for supporting (holding, fixing) the plurality of guide elements are provided. However, different to the above embodiment, the supporting means are preferably not fixed to or mounted on the door due to the pivoting movement of the door. Instead, the supporting means are preferably adapted to be removably provided or mounted within the inside of the tank such that the guide elements can be removed from the tank by removing the supporting means while the guide elements are supported on the supporting means. A corresponding tool for removing the supporting means may be provided.

According to a further alternative embodiment (not shown), the supporting means are fixedly provided within the tank and the guide elements are removed from the tank without also removing the supporting means. In this case, preferably, an additional removing tool may be provided for removing the guide elements.

Additional embodiments of the present teachings include, but are not limited to:
1. A bioreactor (1), such as a photobioreactor, comprising:
   a tank (10) defining an interior volume for holding a growth medium (12), and
   at least a first guide element (20), a second guide element (22), and a third guide element (24) removably mounted or held within the interior volume of the tank and configured such that the guide elements (20, 22, 24) are at least partially immersed, e.g., completely immersed, in the growth medium (12) when the tank (10) is filled with growth medium (12) up to an upper threshold level (L1),
   wherein:
      the first, the second and the third guide elements (20, 22, 24) are disposed side-by-side in a longitudinal direction (X) of the tank (10) to define a first fluid flow chamber (26) between the first and the second guide elements (20, 22) and a second fluid flow chamber (28) between the second and the third guide elements (22, 24),
      the first, the second and the third guide elements (20, 22, 24) are configured such that first and second fluid flow paths (30, 32) for the growth medium (12) are respectively defined in a vertical direction (Y), which is perpendicular to the longitudinal direction, through the first and second fluid flow chambers (26, 28),
      each of the first and second fluid flow paths (30, 32) has a shape or contour in the vertical direction selected from the group consisting of a meandering shape, a zigzag shape, a waveform shape, an undulating shape or a sinusoidal shape, and
      the first, the second and the third guide elements (20, 22, 24) are configured to be detachable from each other at least when removed from the tank (10).
2. The bioreactor according to above embodiment 1, wherein:
   the first guide element (20) comprises, at least on a side that partially defines the first fluid flow chamber (26), a plurality of protrusions (42) that each project or extend towards the second guide element (22) in the longitudinal direction (Z),
   the second guide element (22) comprises, on a first side defining the first fluid flow chamber (26), a first plurality of protrusions (44) that each project or extend towards the first guide element (20) in the longitudinal direction (Z) and, on a second side defining the second fluid flow chamber (28), a second plurality of second protrusions (46) that each project or extend towards the third guide element (24) in the longitudinal direction (Z), and
   the third guide element (24) comprises, at least on a side defining the second flow chamber (28), a plurality of protrusions (48) that each project or extend towards the second guide element (22) in the longitudinal direction (Z),
   wherein:
      the protrusions (42, 44, 46, 48) optionally all have the same shape at least in a vertical cross-section,
      the protrusions (42, 44, 46, 48) optionally are configured as baffles, peaks, ridges, flanges, fins or substantially triangular arches having concave sides,
      each of the protrusions (42, 44, 46, 48) preferably extends entirely, or substantially entirely, across the respective guide element in a lateral direction (Z) that is perpendicular to the longitudinal direction and the vertical direction (Y),
      the protrusions (42, 44) of the first and the second guide elements (20, 22) that together define the first fluid flow chamber (26) are, in the vertical direction (Y), disposed on the first and the second guide elements (20, 22) in an alternating manner to define the first fluid flow path (30) through the first fluid flow chamber (26), and
      the protrusions (46, 48) of the second and the third guide elements (22, 24) that together define the second flow chamber (28) are, in the vertical direction (Y), disposed on the second and the third guide elements (22, 24) in an alternating manner to define the second fluid flow path (32) through the second fluid flow chamber (28).
3. The bioreactor according to the above embodiment 2, wherein:
   the first guide element (20) comprises (is constituted by) a first plate (50) attached to a second plate (52) that is partially parallel to the first plate (50), the second plate (52) partially defining the first fluid flow chamber (26) and comprising the protrusions (42) which were preferably formed by thermoforming, and/or
   the second guide element (22) comprises (is constituted by) a first plate (54) attached to a second plate (56) that is partially parallel to the first plate (54), the second plate (56) partially defining the second flow chamber (28), the first plate (54) of the second guide element (22) having the protrusions (44) and the second plate (56) of the second guide element (22) having the protrusions (46) which also were preferably formed by thermoforming, and/or
   the third guide element (24) comprises a first plate (58) attached to a second plate (60) that is partially parallel to the first plate (58), the second plate partially defining the second flow chamber (28) and comprising the protrusions (48) which also were preferably formed by thermoforming.
4. The bioreactor according to any one of the above embodiments 1 to 3, wherein:
   the guide elements (20, 22, 24) are disposed side-by-side as an array in the longitudinal direction (X) in the following sequence: the first guide element (20), at least two intermediate guide elements (22i), two of which are the second and third guide elements (22, 24), and a last guide element (24i), and
   a third flow chamber (28i) is defined in the vertical direction (Y) between the last guide element (24i) and an adjacent-most one of the intermediate guide elements (22i).
5. The bioreactor according to the above embodiment 4, wherein:
   both sides of each of the intermediate guide elements (22i) comprise (the) protrusions (44, 46) projecting or extending towards an adjacent one of the guide elements in the longitudinal direction (X),
   a first side of the last guide element (24i) that faces the adjacent-most one of the intermediate guide elements (22i) comprises a plurality of protrusions (42) protruding towards the adjacent one of the intermediate guide elements (22i),
   the protrusions (42) of the last guide element (24i) optionally all have the same shape at least in a vertical cross-section as the other protrusions (42, 44, 46) of the other guide elements,
   the protrusions (42) of the last guide element (24i) optionally are configured as baffles, peaks, ridges, flanges, fins or substantially triangular arches having concave sides,
   each of the protrusions (42) of the last guide element (24i) preferably extends entirely, or substantially entirely, across the last guide element (24i) in a (the) lateral direction (Z) that is perpendicular to the longitudinal direction and the vertical direction (Y), and
   flow paths (30, 32, 32i), two of which are the first and second flow paths, are respectively defined between the first guide element (20) and an adjacent one of the intermediate guide elements (22i), between each pair of adjacent ones of the intermediate guide elements (22i), and between the first side of the last guide element (24i) and an adjacent one of the intermediate guide elements (22i), each of the flow paths (30, 32, 32i) having a waveform shape, a meandering shape, a zigzag shape, an undulating shape or a sinusoidal shape.
6. The bioreactor according to any one of the above embodiments 1 to 5, wherein:
   the first and second fluid flow chambers are first upward flow chambers (26, 28, 28i, 28ii) configured to guide an upward flow of the growth medium through the interior volume of the tank (10),
   at least one downward flow chamber (62, 64) is defined within the tank between at least one of the guide elements (20, 22, 22i, 24) and an inside surface (66) of a side wall (68) of the tank or is defined by at least one of the fluid flow chambers, the at least one downward flow chamber (62, 64) being configured to guide a downward flow of the growth medium through the interior volume of the tank (10),
   an open upper end and an open lower end of each of the first upward flow chambers (26, 28, 28i, 28ii) are in fluid communication with the interior volume of the tank,
   the open upper ends of the first upward flow chambers (26, 28, 28i, 28ii) are in fluid communication with the at least one downward flow chamber (62, 64), and the at least one downflow chamber (62, 64) is in fluid communication with the lower ends of the first upward flow chambers (26, 28, 28i, 28ii) to define a closed loop for circulating the growth medium (12) within the tank (10) from the open, upper ends of the first upward flow chambers (26, 28, 28i, 28ii) to the open, lower ends of the first upward flow chambers (26, 28, 28i, 28ii) via the at least one downward flow chamber (62, 64) and from the open lower ends of the first upward flow chambers (26, 28, 28i, 28ii) to the open upper ends of the first upward flow chambers (26, 28, 28i, 28ii) via the first upward flow chambers (26, 28, 28i, 28ii).
7. The bioreactor according to the above embodiment 6, wherein:
   the at least one downward flow chamber comprises at least a first downward flow chamber (62) defined by an inside surface (66a) of a first tank side wall (68a) that extends perpendicular to the longitudinal direction (X) and a surface of the first guide element (20) that faces away from the first fluid flow chamber (26), and/or
   a second upward flow chamber (70) is defined between an inside surface (66b) of a second tank side wall (68b) that extends perpendicular to the longitudinal direction (X) and a surface of the third or last guide element (24, 24i) that faces away from the corresponding chamber (28, 28i) defined by the third or last guide element (24, 24i).
8. The bioreactor according to the above embodiment 6 or 7, wherein:
   at least two of the guide elements (20, 22, 22i, 24, 24i), which together define at least one of the fluid flow chambers (26, 28, 28i, 28ii) therebetween, are physically connected to each other in the longitudinal direction (X) by two connection side walls (72) disposed at lateral ends of the at least two guide elements in a (the) lateral direction (Z), which is perpendicular to the longitudinal direction (X) and to the vertical direction (Y),
   the two connection side walls (72) each extend in the vertical direction from a lower end portion to an upper end portion of the guide elements (20, 22, 22i, 24, 24i),
   in the lateral direction (Z), the two connection side walls (72) partially define or bound ends of the at least one fluid flow chamber (26, 28, 28i, 28ii) that is defined by the physically connected at least two of the guide elements, and
   the at least one downward flow chamber comprises at least a second downflow chamber (64) defined between at least one of the two connection side walls (72) and an inside surface (66) of a third tank side wall (68) extending perpendicular to the lateral direction (Z).
9. The bioreactor according to any one of the above embodiments 1 to 8, further comprising:
   a light emitting means (74) disposed within and/or on at least one of the guide elements (20, 22, 22i, 24, 24i),
   wherein:
      the corresponding guide element(s) (20, 22, 22i, 24, 24i) and the light emitting means (74) are configured such that the light emitting means (74) radiates lights to (illuminates) the growth medium (12) in the fluid flow chambers (26, 28, 28i, 28ii), and
      the light emitting means (74) is preferably disposed inside at least one of the protrusions (42, 44, 46, 48).
10. The bioreactor according to any one of the above embodiments 1 to 9, wherein:
   the tank (10) comprises a bottom wall (76), and
   a pressurized gas chamber (77) is defined in the tank (10),
   wherein:
      a bottom wall of the pressurized gas chamber (77) is integrally formed with the bottom wall (76) of the tank (10),
      an upper wall (78) of the pressurized gas chamber (77) comprises a membrane (80) and/or at least one opening (82) configured to enable pressurized gas within the pressurized gas chamber (77) to enter (permeate, be introduced) into the interior volume of the tank (10), and
      the membrane (80) and/or the at least one opening (82) is (are) configured and disposed such that, when the tank (10) contains the growth medium (12), pressurized gas entering the interior volume of the tank (10) rises into the at least one of the fluid flow chambers (26, 28, 28i, 28ii) and thereby upwardly lifts (moves, pushes) the growth medium (12) within the respective flow chamber(s) (26, 28, 28i, 28ii).
11. The bioreactor according to the above embodiment 10, wherein the upper wall (78) of the pressure gas chamber (77) is configured as a partition wall (78) that divides the tank (10) in the vertical direction (Y) into a first tank volume configured to contain the growth medium (12) and the guide elements, and a second tank volume located below the first tank volume and configured to contain the pressurized gas chamber (77).
12. The bioreactor according to any one of the above embodiments 1 to 11, wherein:
   the tank comprises a (the) bottom wall,
   the bottom wall at least partly defines a (the) pressurized gas chamber defined below the tank,
   the bottom wall has a (the) membrane and/or (the) at least one opening configured to enable pressurized gas on the side opposite to the tank to enter into the interior volume of the tank, and
   the membrane and/or the at least one opening is (are) configured and disposed such that, when the tank contains the growth medium, the pressurized gas entering the interior volume of the tank rises into the at least one of the fluid flow chambers and thereby upwardly lifts the growth medium within the respective flow chamber(s).
13. The bioreactor according to the above embodiment 12, wherein the membrane (80) is removably inserted into a membrane support opening in the corresponding wall or the at least one opening (82) is formed in an insert (86) which is removably inserted into an insert opening in the corresponding wall (76), the at least one opening (82) being preferably defined as a plurality of micro holes extending through the insert (86).
14. The bioreactor according to any one of the above embodiments 1 to 13, wherein:
   the tank (10) comprises at least one removable outer wall portion (88), which is preferably configured as a door or panel that defines one lateral side of the tank (10), and
   a) an inner side of the removable outer wall portion (88) comprises supporting means (90) for supporting the guide elements (20, 22, 22i, 24, 24i), such that the guide elements (20, 22, 22i, 24, 24i) are removable from the tank (10) together with the removeable wall portion (88) when the removable wall portion (88) is removed (detached) from the tank (10), or
   b) the door or panel is hinged to one of the other walls of the tank and supporting means for supporting the guide elements in a mounted state within the tank are provided within the tank such that such that the guide elements are removable from the tank (10) together with the supporting means in a state where guide elements are at least partly supported by the supporting means.
15. The bioreactor according to any one of the above embodiments 1 to 14, further comprising:
   a downpipe (92) fluidly connecting an upper volume of the tank (10) with a lower volume of the tank (10),
   wherein an upper portion of the downpipe (92) is connected to the tank (10) at a location that is displaced in the longitudinal direction (X) and/or in a (the) lateral direction (Z) from a connection of a lower portion of the downpipe (92) to the tank (10).
16. The bioreactor according to any one of the above embodiments 1 to 15, further comprising:
   a heat exchanger (94) disposed at least at a lower region of at least one of the tank side walls (68c, 68d).
17. The bioreactor according to any one of the above embodiments 1 to 16, wherein:
   a first vertical plane extending through planar portions of the first guide element (20) is spaced apart from a second vertical plane extending through planar portions of the second guide element (22) in the longitudinal direction (X) by a first distance (d1); and
   the protrusions (42, 44) of the first and the second guide elements (20, 22) each have a height (h2) in the longitudinal direction (X) that is more than 50% of the first distance (d1), e.g. more than 60%, e.g., more than 70%, e.g., more than 80%, e.g., less than 95%, e.g., less than 90%, e.g., less than 85% or within any range arbitrarily defined by the said upper and lower limits of the height (h2) of the protrusions (42, 44).
18. The bioreactor according to any one of the above embodiments 1 to 17, wherein:
   a third vertical plane extending through planar portions of the third guide element (24) is spaced apart from a (the) second vertical plane extending through planar portions of the second guide element (22) in the longitudinal direction (X) by a (the) first distance (d1); and
   the protrusions (44, 46, 48) of the second and the third guide elements (22, 24) each have a (the) height in the longitudinal direction (X) that is more than 50% of the first distance (d1), e.g. more than 60%, e.g., more than 70%, e.g., more than 80%, e.g., less than 95%, e.g., less than 90%, e.g., less than 85% or within any range arbitrarily defined by the said upper and lower limits of the height of the protrusions (44, 46, 48).
19. A guide element for guiding an upward flow of a growth medium in a bioreactor, comprising:
   a first plate (54), and
   a second plate (56),
   wherein:
      the first plate (54) is fixedly connected to the second plate (56), e.g., by plastic welding or adhesive, and at least planar portions of the first plate are parallel to planar portions of the second plate,
      the first plate (54) comprises protrusions (44) projecting in a longitudinal direction (X) away from the second plate (56) and extending in a lateral direction (Z), which is perpendicular to the longitudinal direction (X), preferably along the entire width of the first plate (54) in the lateral direction (Z), and
      the second plate (56) comprises protrusions (46) projecting in the longitudinal direction (X) away from the first plate (54) and extending in the lateral direction (Z), preferably along the entire width of the second plate (56) in the lateral direction (Z).
20. The guide element according to above embodiment 19, wherein:
   each of the protrusions (44, 46) defines a protrusion cavity (45) configured to house a light emitting means (74), and/or
   the guide element is symmetric along a symmetry axis, which extends in a vertical direction (Y) that is perpendicular to the longitudinal direction (X) and the lateral direction (Z), between the first and the second plates (54, 56) such that longitudinally adjacent protrusions (44, 46) define a common protrusion cavity (45) configured to house a light emitting means (74), and/or
   connection side walls (72) are provided on each of the first and the second plates (54, 56) and protrude in a direction away from the other plate and extend in a vertical direction (Y), which is perpendicular to the longitudinal and the lateral directions, and
   optionally, the connection side walls (72) protrude by a height (h1) in the longitudinal direction (X) which is shorter than a height (h2) of the protrusions (44, 46) in the longitudinal direction (X), and optionally, the connection side walls (72) define a connection side wall cavity (75).

It is explicitly stated that all features disclosed in the description and/or the claims are intended to be disclosed separately and independently from each other for the purpose of original disclosure as well as for the purpose of restricting the claimed invention independent of the composition of the features in the embodiments and/or the claims. It is explicitly stated that all value ranges or indications of groups of entities disclose every possible intermediate value or intermediate entity for the purpose of original disclosure as well as for the purpose of restricting the claimed invention, in particular as limits of value ranges.

### List of reference signs

- 1: bioreactor
- 10: tank
- 12: growth medium
- 20: first guide element
- 22: second guide element
- 22i: intermediate guide element(s)
- 24: third guide element
- 24i: last guide element
- 26: first flow chamber
- 28: second flow chamber
- 28i, 28ii: third and further flow chamber(s)
- 30, 32, 32i: flow path
- 34, 36, 38, 40: surface
- 42, 44, 46, 48: protrusion portions
- 43: (first type) protrusion portion cavity
- 45: (second type) protrusion portion cavity
- 47: plane portion
- 50, 54, 58: first plates
- 52, 56, 60: second plates
- 62: first type downflow chamber
- 64: second type downflow chamber
- 66a, b, c, d: inside surface of the tank
- 68a: front side wall
- 68b: rear side wall
- 68c: lateral side wall
- 68d: lateral side wall
- 70: second type upflow chamber
- 72: connection side elements
- 74: light emitting means
- 75: connection side wall cavity
- 76: bottom wall
- 77: pressure gas chamber
- 78: partition wall
- 80: membrane
- 82: opening
- 84: pressure gas
- 86: insert
- 88: removable (outer) wall portion (door)
- 90: supporting means (bars)
- 91: moving means
- 92: downcomer tube
- 94: heat sink portions
- 96: foam separator
- 98: upper wall

## Claims

1. A bioreactor (1), preferably a photobioreactor, comprising
a tank (10) adapted to contain a growth medium (12), and
a plurality of guide elements mounted within the tank and comprising at least a first guide element (20), a second guide element (22), and a third guide element (24),
wherein
the guide elements (20, 22, 24) are provided within the tank (10) such that the guide elements (20, 22, 24) are at least partially immersed in the growth medium (12) when the tank (10) contains the growth medium (12),
the first, the second and the third guide elements (20, 22, 24) are disposed next to each other in a longitudinal direction (X) such that a first flow chamber (26) is defined between the first and the second guide elements (20, 22) and a second flow chamber (28) is defined between the second and the third guide elements (22, 24), the first, the second and the third guide elements (20, 22, 24) being defined and disposed such that first and second flow paths (30, 32) of the growth medium (12) in a vertical direction (Y) through the respective first and second flow chambers (26, 28) have a waveform shape, a meandering shape, a zigzag shape, an undulating shape or a sinusoidal shape,
the first, the second and the third guide elements (20, 22, 24) are removably mounted within the tank (10) such that the interior of the tank (10) is cleanable when the guide elements (20, 22, 24) have been removed, and
the first, the second and the third guide elements (20, 22, 24) are configured to be separable from each other at least when removed from the tank (10) such that surfaces (34, 36, 38, 40) of the guide elements (20, 22, 24) defining the flow chambers (26, 28) in the mounted state are cleanable.

2. The bioreactor according to claim 1, **characterized in that**
the first guide element (20) comprises, at least on a side partially defining the first flow chamber (26), a plurality of protrusion portions (42) respectively protruding towards the second guide element (22),
the second guide element (22) comprises, on a first side partially defining the first flow chamber (26), a plurality of first protrusion portions (44) protruding towards the first guide element (20) and, on a second side partially defining the second flow chamber (28), a plurality of second protrusion portions (46) protruding towards the third guide element (24), and
the third guide element (24) comprises, at least on a side partially defining the second flow chamber (28), a plurality of protrusion portions (48) respectively protruding towards the second guide element (22),
wherein:
the protrusion portions (42) of the first guide element (20) and the first protrusion portions (44) of the second guide element (22) that together define the first flow chamber (26) are, in the vertical direction (Y), disposed on the first and the second guide elements (20, 22) in an alternating manner to define the first flow path (30) through the first flow chamber (26), and
the second protrusion portions (46) of the second guide element (22) and the protrusion portions (48) of the third guide element (24) that together define the second flow chamber (28) are, in the vertical direction (Y), disposed on the second and the third guide elements (22, 24) in an alternating manner to define the second flow path (32) through the second flow chamber (28).

3. The bioreactor according to claim 2, **characterized in that**
the first guide element (20) comprises a first plate (50) attached to a second plate (52) that is partially parallel to the first plate (50), the second plate (52) partially defining the first flow chamber (26) and comprising the protrusion portions (42) which are preferably formed by thermoforming, and/or
the second guide element (22) comprises a first plate (54) attached to a second plate (56) that is partially parallel to the first plate (54), the first plate (54) partially defining the first flow chamber (26) and the second plate (56) partially defining the second flow chamber (28), the first plate (54) of the second guide element (22) having the first protrusion portions (44) and the second plate (56) of the second guide element (22) having the second protrusion portions (46) which are preferably formed by thermoforming, and/or
the third guide element (24) comprises a first plate (58) attached to a second plate (60) that is partially parallel to the first plate (58), the first plate (58) partially defining the second flow chamber (28) and comprising the protrusion portions (48) which are preferably formed by thermoforming.

4. The bioreactor according to any one of claims 1 to 3, **characterized in that**
the guide elements (20, 22, 24) are disposed next to each other as an array in the longitudinal direction (X), which is perpendicular to the vertical direction (Y), and comprise, in the longitudinal direction (X), in the following sequence, the first guide element (20), at least two intermediate guide elements (22i), two of which are the second and third guide elements (22, 24), and a last guide element (24i) in the longitudinal direction (X), and
a third flow chamber (28ii) is defined between the last guide element (24i) and an adjacent one of the intermediate guide elements (22i).

5. The bioreactor according to claim 4, **characterized in that**
both sides of each of the intermediate guide elements (22i) comprise a plurality of protrusion portions (44, 46, 48, 60) protruding towards an adjacent one of the guide elements, and a side of the last guide element (24i) facing the adjacent one of the intermediate guide elements (22i) comprises a plurality of protrusion portions protruding towards the adjacent one of the intermediate guide elements (22i) such that flow paths (30, 32, 32i), two of which are the first and second flow paths, are respectively defined between the first guide element (20) and an adjacent one of the intermediate guide elements (22i), between each pair of adjacent ones of the intermediate guide elements (22i), and between the side of the last guide element (24i) and an adjacent one of the intermediate guide elements (22i), each of the flow paths (30, 32, 32i) having a waveform shape, a meandering shape, a zigzag shape, an undulating shape or a sinusoidal shape.

6. The bioreactor according to any one of claims 1 to 5, **characterized in that**
at least two of the flow chambers are first type upflow chambers (26, 28, 28i, 28ii) which are adapted to guide an upflow of the growth medium,
at least one downflow chamber (62, 64) adapted to guide a downflow of the growth medium is defined within the tank between at least one of the guide elements (20, 22, 22i, 24) and an inside surface (66) of a side wall (68) of the tank or is defined by at least one of the flow chambers, and
an upper end portion and a lower end portion of each of the first type upflow chambers (26, 28, 28i, 28ii) are fluidly connected with the interior volume of the tank, and
upper end portions of the first type upflow chambers (26, 28, 28i, 28ii) are in fluid communication with the at least one downflow chamber (62, 64) and the at least one downflow chamber (62, 64) is in fluid communication with lower end portions of the first type upflow chambers (26, 28, 28i, 28ii) to circulate the growth medium (12) within the tank (10) from the upper end portions of the first type upflow chambers (26, 28, 28i, 28ii) to the lower end portions of the first type upflow chambers (26, 28, 28i, 28ii) via the at least one downflow chamber (62, 64) and from the lower end portions of the first type upflow chambers (26, 28, 28i, 28ii) to the upper end portions of the first type upflow chambers (26, 28, 28i, 28ii) via the first type upflow chambers (26, 28, 28i, 28ii).

7. The bioreactor according to claim 6, **characterized in that**
the at least one downflow chamber comprises at least a first type downflow chamber (62) defined by an inside surface (66a) of a first tank side wall (68a) extending perpendicular to the longitudinal direction (X) and a surface of the first guide element (20) facing away from the first flow chamber (26), and/or
a second type upflow chamber (70) is defined between an inside surface (66b) of a second tank side wall (68b) extending perpendicular to the longitudinal direction (X) and a surface of the third or last guide element (24, 24i) facing away from the corresponding chamber (28, 28i) defined by the third or last guide element (24, 24i).

8. The bioreactor according to claim 6 or 7, **characterized in that**
at least two of the guide elements (20, 22, 22i, 24, 24i), which together form one of the flow chambers (26, 28, 28i, 28ii) therebetween, are physically connected to each other by two connection side walls (72) respectively extending along lateral edge portions of the at least two of the guide elements (20, 22, 22i, 24, 24i) in the vertical direction (Y) from a lower end portion to an upper end portion of the at least two of the guide elements (20, 22, 22i, 24, 24i) and also projecting in the longitudinal direction (X), and
the at least one downflow chamber comprises at least a second type downflow chamber (64) defined between at least one of the connection side walls (72) and an inside surface (66) of a third tank side wall (68) extending perpendicular to a lateral direction (Z) that is perpendicular to the longitudinal direction (X) and to the vertical direction (Y).

9. The bioreactor according to any one of claims 1 to 8, **characterized by** further comprising
a light emitting means (74) provided within or on at least one of the plurality of guide elements (20, 22, 22i, 24, 24i),
wherein the corresponding guide element(s) (20, 22, 22i, 24, 24i) and the light emitting means (74) are configured such that the growth medium (12) in the flow chambers (26, 28, 28i, 28ii) can be irradiated with light, and
the light emitting means (74) is preferably provided inside at least one of the protrusion portions (42, 44, 46, 48).

10. The bioreactor according to any one of claims 1 to 9, **characterized in that**
the tank (10) comprises a bottom wall (76), and
a pressurized gas chamber (77) is defined in the tank (10),
wherein
a bottom wall of the pressurized gas chamber (77) is integrally formed with the bottom wall (76) of the tank (10),
an upper wall (78) of the pressurized gas chamber (77) comprises a membrane (80) and/or at least one opening (82) adapted to enable pressurized gas within the pressurized gas chamber (77) to enter into the tank (10), and
the membrane (80) and/or the at least one opening (82) is (are) adapted and provided such that, when the tank (10) contains the growth medium (12), pressurized gas (84) entering the interior volume of the tank (10) rises into the at least one of the flow chambers (26, 28, 28i, 28ii) and thereby upwardly lifts the growth medium (12) within the respective flow chamber(s) (26, 28, 28i, 28ii).

11. The bioreactor according to claim 10, **characterized in that**
the upper wall of the pressure gas chamber (77) is formed as a partition wall (78) dividing the tank (10) in the vertical direction (Y) into a first tank portion, which is adapted to contain the growth medium (12) and the guide elements, and a second tank portion, which is located below the first tank portion and defines the pressurized gas chamber (77).

12. The bioreactor according to any one of claims 1 to 9, **characterized in that**
the tank comprises a bottom wall,
the bottom wall at least partly defines a pressurized gas chamber defined below the tank,
the bottom wall has a membrane and/or at least one opening adapted to enable pressurized gas on the side opposite to the tank to enter into the tank, and
the membrane and/or the at least one opening is (are) adapted and provided such that, in case the tank contains the growth medium, the pressurized gas entering the interior volume of the tank rises into the at least one of the flow chambers and thereby upwardly lifts the growth medium within the respective flow chamber(s).

13. The bioreactor according to claim 12, **characterized in that**
the membrane (80) is removably inserted into a membrane support opening in the corresponding wall or the at least one opening (82) is formed in an insert (86) which is removably inserted into an insert opening in the corresponding wall (76), the at least one opening (82) being preferably defined as a plurality of micro holes extending through the insert (86).

14. The bioreactor according to any one of claims 1 to 13, **characterized in that**
the tank (10) comprises at least one removable outer wall portion (88), preferably a door, and
optionally, an inner side of the removable outer wall portion (88) comprises supporting means (90) for supporting the plurality of guide elements (20, 22, 22i, 24, 24i), such that the plurality of guide elements (20, 22, 22i, 24, 24i) is removed from the tank (10) together with the removeable wall portion (88) when the removable wall portion (88) is removed from the tank (10).

15. The bioreactor according to any one of claims 1 to 14, **characterized by** further comprising
a downcomer tube (92) fluidly connecting an upper portion of the tank (10) with a lower portion,
wherein a connection of an upper portion of the downcomer tube (92) to the tank (10) is located at an opposite position in the longitudinal direction (X) and/or at an opposite position in the lateral direction (Z) relative to the connection of a lower portion of the downcomer tube (92) to the tank (10).

16. The bioreactor according to any one of claims 1 to 15, **characterized by** further comprising
a heat sink portion (94) provided at least at or on a lower region of at least one of the tank side walls (68c, 68d).

17. A guide element for guiding an upward flow of a growth medium in a bioreactor, comprising
a first plate (54), and
a second plate (56),
wherein
the first plate (54) is fixedly connected and partially parallel to the second plate (56),
the first plate (54) comprises protrusion portions (44) protruding in a longitudinal direction (X) away from the second plate (56) and extending in a lateral direction (Z), which is perpendicular to the longitudinal direction (X), and
the second plate (56) comprises protrusion portions (46) protruding in a direction away from the first plate (54) and extending in the lateral direction (Z).

18. The guide element according to claim 17, **characterized in that**
each protrusion portion (44, 46) defines a protrusion portion cavity (45), which cavity (45) is adapted to house a light emitting means (74), and/or
the guide element is symmetric with its symmetry axis between the first and second plates (54, 56) such that longitudinally-adjacent protrusion portions (44, 46) form a common protrusion portion cavity (45) adapted to house a light emitting means (74), and/or
connection side walls (72) are provided on each plate (54, 56) and protrude in a direction away from the other plate and extend in a vertical direction (Y), which is perpendicular to the longitudinal and the lateral directions, and,
optionally, the connection side walls (72) protrude by a height (h1) in the longitudinal direction (X) which is shorter than a height (h2) of the protrusion portions (44, 46) in the longitudinal direction (X), and optionally, the connection side walls (72) form a connection side wall cavity (75).
